(19) Europäisches Patentamt
European Patent Office
Office européen des brevets

(11) Publication number: **0 377 295 B1**

# (12) EUROPEAN PATENT SPECIFICATION

(45) Date of publication of patent specification: **01.02.95**

(51) Int. Cl.[6]: **C12N 15/61**, C12N 9/90, C12N 1/21, C12N 1/15, //(C12N1/21,C12R1:19), (C12N1/15,C12R1:465), (C12N1/15,C12R1:66), (C12N1/15,C12R1:745), (C12N1/15,C12R1:80)

(21) Application number: **89313150.8**

(22) Date of filing: **15.12.89**

## (54) Recombinant DNA expression vectors and DNA compounds that encode isopenicillin N epimerase (racemase) activity.

(30) Priority: **22.12.88 US 288760**

(43) Date of publication of application:
**11.07.90 Bulletin 90/28**

(45) Publication of the grant of the patent:
**01.02.95 Bulletin 95/05**

(84) Designated Contracting States:
**AT BE CH DE ES FR GB GR IT LI NL SE**

(56) References cited:
**EP-A- 0 233 175**

**BIOCHIM. BIOPHYS. ACTA, 1989, vol. 999, no. 1, pages 78-85; S. USUI et al.:"Purification and properties of isopenicillin N epimerase from Streptomycesclavuligerus"**

(73) Proprietor: **ELI LILLY AND COMPANY**
**Lilly Corporate Center**
**Indianapolis**
**Indiana 46285 (US)**

(72) Inventor: **Kovacevic, Steven**
**5620 Broadway**
**Indianapolis**
**Indiana 46220 (US)**
Inventor: **Miller, James Robert**
**9244 Thrushwood Lane**
**Indianapolis**
**Indiana 46250 (US)**
Inventor: **Skatrud, Paul Luther**
**1045 Fiesta Drive**
**Greenwood**
**Indiana 46142 (US)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 377 295 B1

CAN. J. MICROBIOL., 1983, vol. 29, pages 1526-1531; S.E. JENSEN et al.:"Partial purification and characterization of isopenicillin N epimeraseactivity from Streptomyces clavuligerus"

Inventor: **Tobin, Matthew Barry**
**3248 Tulip Drive**
**Indianapolis**
**Indiana 46227 (US)**

(74) Representative: **Hudson, Christopher Mark et al**
**Lilly Industries Limited**
**European Patent Operations**
**Erl Wood Manor**
**Windlesham**
**Surrey GU20 6PH (GB)**

## Description

The natural sulfur-containing β-lactam antibiotics are the most important clinically, and the isolation of novel β-lactam compounds continues nearly six decades since the discovery of the penicillins by Fleming. The common structural feature of the penicillins and cephalosporins (including cephamycins) is the β-lactam ring.

These antibiotics are produced by a variety of prokaryotes and lower eukaryotes. The penicillins, exemplified by the compounds penicillin G (benzylpenicillin) or penicillin V, are produced mainly by filamentous fungi, most notably Penicillium chrysogenum. The cephalosporins, first isolated as a product from the lower eukaryote, Cephalosporium acremonium (syn. Acremonium chrysogenum), are also metabolites of many prokaryotes but especially Streptomyces clavuligerus, S. lipmanii and S. cattleya that also produce cephamycins and other β-lactams such as oxypenams (clavulanic acid) and carbapenams (thienamycin).

The development of cell-free systems from β-lactam-producing organisms has allowed the establishment of the biosynthetic steps in the pathway of the sulfur-containing β-lactams (penicillins and cephalosporins).

The initial steps in the formation of penicillins in filamentous fungi (e.g. P. chrysogenum), and the cephalosporins and cephamycins produced by both prokaryotes and lower eukaryotes, are identical. ACV synthetase catalyzes the condensation of the amino acid precursors L-α-aminoadipate, L-cysteine, and L-valine to the tripeptide LLD-ACV. The next step forms the first β-lactam in the pathway by the cyclization of the tripeptide yielding isopenicillin N (IPN), the precursor to all penicillins, cephalosporins and cephamycins.

After synthesis of IPN, the pathways to cephalosporins and penicillins diverge. In Penicillium chrysogenum, for example, the α-aminoadipyl side chain of IPN can be exchanged for one of many (nearly 100 to date) hydrophobic side chains derived from the corresponding acyl CoA. One of the most familiar examples is the formation of penicillin G (benzylpenicillin) from phenylacetyl CoA and IPN. However, in the fungus C. acremonium, the α-aminoadipyl side chain is isomerized to produce penicillin N. The five-membered thiazolidine ring of the penicillin is then "expanded" to the six-membered dihydrothiazine ring that is characteristic of the cephalosporins. This reaction is catalyzed by deacetoxycephalosporin C synthetase (DAOCS) and produces the first cephalosporin in the pathway, deacetoxycephalosporin C (DAOC). Although the DAOCS activities from Cephalosporium acremonium and the gram-positive bacteria, Streptomyces clavuligerus and S. lactamdurans, have been extensively characterized, the remaining steps in the pathway are less well understood.

The present invention provides a gene encoding an activity of the β-lactam biosynthetic pathway, isopenicillin N epimerase. The activity is also known as racemase. The present invention expands the repertoire of beta-lactam biosynthetic enzymes which can be over-produced. This ability facilitates the bioconversion of substrate analogs to novel beta-lactams and strain improvement by increased gene dosage.

The present invention comprises a DNA sequence that encodes isopenicillin N epimerase activity from Streptomyces clavuligerus. Isopenicillin N epimerase (racemase) catalyzes the reaction in which penicillin N is formed from isopenicillin N and is also referred to as racemase. The enzyme converts the L-alpha-aminoadipyl side chain of isopenicillin N to a D-alpha-aminoadipyl side chain. It is now referred to as racemase rather than the former epimerase because it appears to establish an equilibrium between the two forms. The enzyme is highly unstable, but the reaction catalyzed is a critical step in the biosynthesis of important antibiotics, such as cephalosporins from Cephalosporium acremonium and 7α-methoxycephalosporins or cephamycins from Streptomyces clavuligerus and S. lipmanii. Thus, it is quite important to be able to produce the activity in large quantities.

The DNA compounds of the present invention encode the racemase activity in a single open reading frame. Transcription of this open reading frame, followed by translation of the resulting mRNA, yields a single polypeptide chain that possesses racemase activity. The DNA compound that encodes the racemase activity was isolated from Streptomyces clavuligerus genomic DNA and used to construct recombinant DNA expression vectors. Four types of these expression vectors are especially useful: the first drives high-level expression of the racemase activity in E. coli; the second in Cephalosporium; the third in Penicillium; and the fourth in Streptomyces.

The E. coli-produced racemase activity catalyzes the formation of penicillin N from isopenicillin N. Crude cell extracts of these E. coli transformants of the invention exhibit racemase activity. These E. coli expression vectors and transformants provide an efficient means for obtaining large amounts of active racemase. The racemase is useful not only for the production of penicillin N but also for the production of unique antibiotics.

The Cephalosporium vectors of the present invention are useful for purposes of constructing strains for use by the pharmaceutical industry. Cephalosporium is an economically important organism used to produce cephalosporin antibiotics. Transformation of Cephalosporium with the expression vectors of this invention results in higher intracellular levels of racemase activity in the transformants. These transformants can be used to increase the efficiency of, and yield of antibiotic in, industrial fermentation processes. Transformation of C. acremonium strains that lack a functional racemase gene with vectors of the invention yields transformants that synthesize penicillin N. Penicillin N is useful as an intermediate in the preparation of orally absorbed, clinically important antibiotics.

The Penicillium vectors of the present invention are most useful to introduce cephalosporin synthesizing activities into high-level penicillin producing Penicillium strains. The racemase activity is useful for the formation of penicillin N as a precursor to the conversion of the various penicillins to cephalosporins by expandase, either alone or in conjunction with other activities, such as deacetylcephalosporin C synthetase (DACS, which catalyzes the formation of deacetylcephalosporin C from DAOC, a hydroxylation reaction). For example, concomitant expression of isopenicillin N epimerase activity and DAOCS activity in Penicillium leads to production of DAOC, a heretofore unknown metabolite in Penicillium.

The DNA compounds encoding the racemase activity are readily modified to construct expression vectors that increase the efficiency and yield of fermentations involving other organisms, such as Paecilomyces and Streptomyces, especially S. clavuligerus. Although the racemase-activity-encoding DNA of the present invention was isolated from S. clavuligerus, this DNA can be used to construct vectors that drive expression of racemase activity in a wide variety of host cells, as illustrated by the E. coli expression vectors described above. The construction protocols utilized for the E. coli, Cephalosporium, and Penicillium vectors of the invention can be followed to construct analogous vectors for other organisms, merely by substituting, if necessary, the appropriate regulatory elements. The racemase-encoding DNA compounds of the present invention can be used to construct expression vectors useful for improving the efficiency and yield of fermentations involving a wide variety of penicillin and cephalosporin antibiotic-producing organisms.

The following section provides a more detailed description of the present invention. For purposes of clarity and as an aid in the understanding of the invention, as disclosed and claimed herein, the following items are defined below.

amdS - an acetamidase gene; also used in the Figures to denote the Aspergillus nidulans acetamidase gene.

amdSp - the promoter of the amdS gene.

AmR - the apramycin resistance-conferring gene; also used to denote the apramycin-resistant phenotype.

Antibiotic - a substance produced by a microorganism that, either naturally or with limited modification, will inhibit the growth of or kill another microorganism or eukaryotic cell.

Antibiotic Biosynthetic Gene - a DNA segment that encodes an activity necessary for an enzymatic reaction in the process of converting primary metabolites into antibiotics or converting one antibiotic compound into a different antibiotic compound.

Antibiotic-Producing Organism - any organism, including, but not limited to, Streptomyces, Bacillus, Flavobacterium, Monospora, Cephalosporium, Paecilomyces, Podospora, Penicillium, and Nocardia, that either produces an antibiotic or contains genes that, if expressed, would produce an antibiotic.

Antibiotic Resistance-Conferring Gene - a DNA segment that encodes an activity that confers resistance to an antibiotic.

ApR - the ampicillin resistance-conferring gene; also used to denote the ampicillin-resistant phenotype.

bGH - bovine growth hormone or DNA encoding same.

bp - a base pair of double-stranded DNA.

CAT - the chloramphenicol resistance-conferring gene, which encodes an acetyltransferase.

cI857 - a gene encoding a temperature sensitive repressor of the λpL promoter.

cIPS - isopenicillin N synthetase or isopenicillin N synthetase-encoding DNA from Cephalosporium acremonium.

Cloning - the process of incorporating a segment of DNA into a recombinant DNA cloning vector.

Coding sequence - the sequence of DNA in a gene that encodes either the amino acid residue sequence of the protein expressed by the gene or, in the case of rRNA or tRNA genes, the RNA sequence of the rRNA or tRNA expressed by the gene.

Cosmid - a recombinant DNA cloning vector that can replicate in a host cell in the same manner as a plasmid but that can also utilize phage packaging mechanisms.

Gene - a segment of DNA that comprises a promoter, translational activating sequence, coding sequence, and 3′ regulatory sequences positioned to drive expression of the gene product, either a protein (and thus necessarily an mRNA), tRNA, or rRNA.

Genomic Library - a set of recombinant DNA cloning vectors into which segments of DNA, which substantially represent the entire genome of a particular organism, have been cloned.

hGH - human growth hormone or DNA encoding same.

HmR - the hygromycin resistance-conferring gene; also used to denote the hygromycin-resistant phenotype.

Hybridization - the process of annealing two single-stranded DNA molecules to form a double-stranded DNA molecule that may or may not be completely base-paired.

IPS or IPNS - isopenicillin N synthetase.

Isopenicillin N - has the structure depicted below:

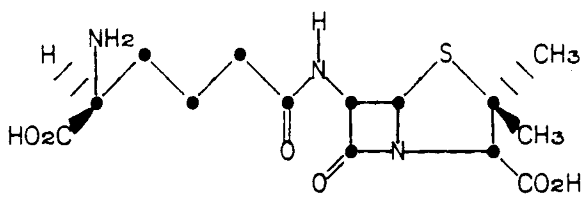

Isopenicillin N Synthetase - an enzyme, also known as cyclase, that catalyzes the formation of isopenicillin N from δ-(L-α-aminoadipyl)-L-cysteinyl-D-valine.

KmR - the kanamycin resistance-conferring gene; also used to denote the kanamycin-resistant phenotype.

lacI - the E. coli lacI gene.

lacZα - the promoter and β-galactosidase (lacZ) α-fragment derived from the E. coli lac operon.

lppT - the transcription terminator of the E. coli lpp gene.

lppP - the promoter of the E. coli lpp gene.

M13 ORI - the origin of replication of phage M13.

mel - the tyrosinase gene.

MCS - a multiple-cloning site.

mRNA - messenger ribonucleic acid.

ORI - a plasmid or vector origin of replication, the DNA sequence that serves as an attachment or start site for DNA polymerase.

Pen DNA - DNA from Penicillium chrysogenum.

Penicillin N - has the structure depicted below:

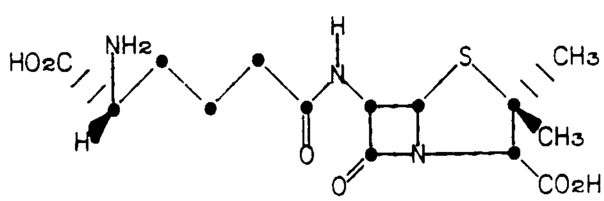

pIPS - the IPS gene or IPS coding sequence of Penicillium chrysogenum.

pIPSp - the promoter of the IPS gene of Penicillium chrysogenum.

pIPSt - the transcription terminator of the IPS gene of Penicillium chrysogenum.

pL - the leftward promoter from bacteriophage lambda.

Promoter - a DNA sequence that directs or initiates the transcription of DNA.

Racemase activity - an enzymatic activity which catalyzes the conversion of isopenicillin N to penicillin N and vice-versa and is encoded by the gene of the present invention or by DNA which can be identified by using the gene of the present invention as a probe.

Recombinant DNA Cloning Vector - any autonomously replicating or integrating agent, including, but not limited to, plasmids, comprising a DNA molecule to which one or more additional DNA molecules can be or

have been added.

Recombinant DNA Expression Vector - any autonomously replicating or integrating agent, including, but not limited to, plasmids, comprising a promoter and other regulatory sequences positioned to drive expression of a DNA segment that encodes a polypeptide or RNA of research or commercial interest.

Recombinant DNA Vector - any recombinant DNA cloning or expression vector.

Restriction Fragment - any linear DNA molecule generated by the action of one or more enzymes.

rRNA - ribosomal ribonucleic acid.

Sensitive Host Cell - a host cell that cannot grow or whose growth is inhibited in the presence of a given antibiotic without a DNA segment that confers resistance thereto.

TcR - the tetracycline resistance-conferring gene; also used to denote the tetracycline-resistant phenotype.

Transcription Terminator - a DNA sequence that signals the termination of transcription of DNA by RNA polymerase.

Transfectant - a recipient host cell that has undergone transformation by phage DNA or by DNA packaged into a phage particle.

Transformant - a recipient host cell that has undergone transformation.

Transformation - the introduction of DNA into a recipient host cell that changes the genotype and results in a change in the recipient cell.

Translational activating sequence - a regulatory DNA sequence that, when transcribed into mRNA, promotes translation of mRNA into protein.

tRNA - transfer ribonucleic acid.

trp - the promoter and translational activating sequence of the tryptophan operon of E. coli.

The restriction site and function maps presented in the accompanying drawings are approximate representations of the recombinant DNA vectors discussed herein. The restriction site information is not exhaustive; therefore, there may be more restriction sites of a given type on the vector than actually shown on the map.

Figure 1. A restriction site and function map of plasmid pOW390.

Figure 2. A restriction site and function map of plasmid pCZR111.

Figure 3. A restriction site and function map of plasmid pOW392.

Figure 4. A diagram of beta-lactam biosynthetic pathways.

Figure 5. A restriction site and function map of plasmid pPSJ78.

Figure 6. A restriction site and function map of plasmid pLC2.

Figure 7. A restriction site and function map of plasmid pPSJ77.

Figure 8. A restriction site and function map of plasmid pPS51.

Figure 9. A restriction site and function map of plasmid pMLC12.

Figure 10. A restriction site and function map of plasmid p3SR2.

The present invention comprises DNA compounds and recombinant DNA cloning and expression vectors that encode the racemase activity of Streptomyces clavuligerus.

In particular the invention comprises a constructed DNA compound that encodes a protein having the isopenicillin N epimerase (racemase) activity of Streptomyces clavuligerus. The sequence of the S. clavuligerus racemase-encoding DNA is depicted below, together with a portion of the DNA that flanks the 3' end of the coding region in the S. clavuligerus genome. In the depiction, only the "sense" or coding strand of the double-stranded DNA molecule is shown, and the DNA is depicted from left to right in the 5' → 3' orientation. The nucleotide sequence is numbered; the numbers appear to the left of the DNA sequence. The amino acid residue sequence of the racemase encoded by the DNA is listed, following the DNA sequence, from left to right in the amino-terminus → carboxy-terminus direction.

```
   1  ATGGCGGTAG CCGACTGGGA AGAAGCCCGC GGCCGTATGC TGCTCGACCC
  51  CACCGTCGTC AACCTCAACA CCGGCTCCGG GGGACCGCTG CCGCGCAGCT
 101  TCGAGCGGGT CACCGGCTTC CGCGCCCATC TCGCGGCCGA GCCGATGGAC
 151  TTCCTGCTCC GCGAGGTCCC CGCACTGCTG TGGCAGGCGC GGGAGAGCCT
 201  CGCCCGCCTC ATCGGCGGGG ACCCGCTGCG CCTCGCCCTG GCCACCAACG
 251  TCACCGCCGC CGTCAACCTC GTCGCGTCGT CACTGCGCCT CGAAGCGCCC
 301  GGCGAGATCC TGCTCAGCGA CGACGAGTAC ACGCCCATGC GCTGGTGCTG
 351  GGAGCGGGTC GCCCGGCGGC ACGGCCTGGA GCTGAGGACG TTCCGGCTGC
 401  CCGAGCTGCC CTCGGACCCG GCCGAGATCA CCGCGGCGGC GGTCGCCGCG
 451  ATGGGACCGC GCACCCGGCT GTTCTTCTTC AGCCATGTCG TCTCCACGAC
 501  CGGGCTGATC CTGCCCGCCG CCGAACTGTG CGAGGAGGCC CGCGCACGGG
 551  GCATCACCAC CGTGGTCGAC GGCGCCCACG CACCCGGCTT CCTCGACCTC
 601  GACCTCTCCC GGATCCCCTG CGACTTCTAC GCGGGCAGCG GCCACAAATG
 651  GCTGCTCGCC CCCACCGGGG TCGGCTTTCT CCACCTCGCC CCCGGCCGCC
 701  TGGAAGAACT GGAGCCCACC CAGGTGAGCT GGGCGTACGA GCCCCCGGAG
 751  GCAGCGGCCC GCCGGCCGCG CGCGACCGCT GTCGGCAGCA CACCCGGGCT
 801  GCGCAGACTC GAATGCGAGG GGACCCGGGA CATCTGCCCC TGGCTCGCCA
 851  CACCGGAGTC GATCGACTTC CAGGCCGAGC TGGGCCCCGG GGCGATCCGC
 901  GCCCGCCGCC GCGAGCTGAC GGACCACGCG CGCCGCCTGC TCGCCGACCG
 951  CCCGGGCCGC ACGCTCCTCA CCCCCGACTC CCCGGAGCTG TCCGGCGGCA
1001  TGGTGGCCTA CCGGCTGCCC CCGGGAACCG ACGCGGCCGA GCTGCGCCGG
1051  GGGCTCTGGG AGCGCTTCCG GATCGAGGCC GCGGTGGCCG AGCAGCCGCC
1101  CGGGCCGGTG CTCCGGATCT CGGCGAACTT CTACACCACC GAAGAGGAGA
1151  TCGACCGCCT GGCGGACGCG CTGGACGCGC TGACGGGCGA GTGATCCCCC
1201  CGGCTCGCGG ACCGCCTCCC CCGCGCTGTT GACCACCCGG TTCACGGATT
1251  ACGAGAGGAT CAGTGAGAGT TG
```

wherein A is deoxyadenyl, G is deoxyguanyl, C is deoxycytidyl, and T is thymidyl.

Following is the amino acid sequence encoded by the preceding DNA sequence:

$NH_2$-Met Ala Val Ala Asp Trp Glu Glu Ala Arg
Gly Arg Met Leu Leu Asp Pro Thr Val Val
Asn Leu Asn Thr Gly Ser Gly Gly Pro Leu
Pro Arg Ser Phe Glu Arg Val Thr Gly Phe
Arg Ala His Leu Ala Ala Glu Pro Met Asp
Phe Leu Leu Arg Glu Val Pro Ala Leu Leu
Trp Gln Ala Arg Glu Ser Leu Ala Arg Leu
Ile Gly Gly Asp Pro Leu Arg Leu Ala Leu
Ala Thr Asn Val Thr Ala Ala Val Asn Leu
Val Ala Ser Ser Leu Arg Leu Glu Ala Pro
Gly Glu Ile Leu Leu Ser Asp Asp Glu Tyr
Thr Pro Met Arg Trp Cys Trp Glu Arg Val
Ala Arg Arg His Gly Leu Glu Leu Arg Thr
Phe Arg Leu Pro Glu Leu Pro Ser Asp Pro
Ala Glu Ile Thr Ala Ala Ala Val Ala Ala
Met Gly Pro Arg Thr Arg Leu Phe Phe Phe
Ser His Val Val Ser Thr Thr Gly Leu Ile
Leu Pro Ala Ala Glu Leu Cys Glu Glu Ala
Arg Ala Arg Gly Ile Thr Thr Val Val Asp
Gly Ala His Ala Pro Gly Phe Leu Asp Leu
Asp Leu Ser Arg Ile Pro Cys Asp Phe Tyr
Ala Gly Ser Gly His Lys Trp Leu Leu Ala
Pro Thr Gly Val Gly Phe Leu His Leu Ala
Pro Gly Arg Leu Glu Glu Leu Glu Pro Thr
Gln Val Ser Trp Ala Tyr Glu Pro Pro Glu
Ala Ala Ala Arg Arg Pro Arg Ala Thr Ala
Val Gly Ser Thr Pro Gly Leu Arg Arg Leu
Glu Cys Glu Gly Thr Arg Asp Ile Cys Pro

Trp Leu Ala Thr Pro Glu Ser Ile Asp Phe
Gln Ala Glu Leu Gly Pro Gly Ala Ile Arg
Ala Arg Arg Arg Glu Leu Thr Asp His Ala
Arg Arg Leu Leu Ala Asp Arg Pro Gly Arg
Thr Leu Leu Thr Pro Asp Ser Pro Glu Leu
Ser Gly Gly Met Val Ala Tyr Arg Leu Pro
Pro Gly Thr Asp Ala Ala Glu Leu Arg Arg
Gly Leu Trp Glu Arg Phe Arg Ile Glu Ala
Ala Val Ala Glu Gln Pro Pro Gly Pro Val
Leu Arg Ile Ser Ala Asn Phe Tyr Thr Thr
Glu Glu Glu Ile Asp Arg Leu Ala Asp Ala
Leu Asp Ala Leu Thr Gly Glu End Ser Pro
Arg Leu Ala Asp Arg Leu Pro Arg Ala Val
Asp His Pro Val His Gly Leu Arg Glu Asp
Gln-COOH

wherein Ala is an alanine residue, Arg is an arginine residue, Asn is an asparagine residue, Asp is an aspartic acid residue, Cys is a cysteine residue, Gln is a glutamine residue, Glu is a glutamic acid residue, Gly is a glycine residue, His is a histidine residue, Ile is an isoleucine residue, Leu is a leucine residue, Lys is a lysine residue, Met is a methionine residue, Phe is a phenylalanine residue, Pro is a proline residue, Ser is a serine residue, Thr is a threonine residue, Trp is a tryptophan residue, Tyr is a tyrosine residue, and Val is a valine residue.

Those skilled in the art will recognize that the DNA sequence depicted above is an important part of the present invention. The above sequence can be conventionally synthesized by the modified phosphotriester method using fully protected deoxyribonucleotide building blocks. Such synthetic methods are well known in the art and can be carried out in substantial accordance with the procedure of Itakura et al., 1977, Science 198:1056 and Crea et al., 1978, Proc. Nat. Acad. Sci. USA 75:5765. In addition, an especially preferred method is disclosed in Hsiung et al., 1983, Nucleic Acid Research 11:3227 and Narang et al., 1980, Methods in Enzymology 68:90. In addition to the manual procedures referenced above, the DNA sequence can be synthesized using automated DNA synthesizers, such as the Systec 1450A or ABS (Applied Biosystems, 850 Lincoln Centre Drive, Foster City, CA 94404) 380A DNA Synthesizers.

Due to the degenerate nature of the genetic code, which results from there being more than one codon for most of the amino acid residues and the translation stop signal, the amino acid residue sequence of racemase enzyme depicted above can be encoded by a multitude of different DNA sequences. Because these alternate DNA sequences would encode the same amino acid residue sequence of the present invention, the present invention further comprises these alternate sequences.

The present invention comprises DNA compounds and recombinant DNA cloning and expression vectors that encode the racemase activity of Streptomyces clavuligerus.

The racemase activity encoding DNA compounds of the present invention were isolated from a strain of Streptomyces clavuligerus. A genomic library of the total genomic DNA of the S. clavuligerus strain was constructed and examined for the presence of sequences homologous to a deoxyribooligonucleotide probe. This probe was constructed in accordance with information obtained about the amino-terminal amino acid sequence of the S. clavuligerus racemase, with knowledge of the genetic code, and with knowledge of codon usage preferences of Streptomyces. DNA sequencing revealed which vector encoded the S. clavuligerus racemase.

An ~3.6 kb KpnI restriction fragment of a cosmid designated as pOW379 that was identified as comprising the racemase gene was inserted into commercially available plasmid pUC19 (digested with KpnI) to yield plasmid pOW390, which was then transformed into E. coli K12 RR1ΔM15 host cells. The E. coli K12 RR1ΔM15/pOW390 transformants have been deposited and made part of the stock culture

collection of the Northern Regional Research Laboratories (NRRL), Peoria, IL 61604, under the accession number NRRL B-18431 (date of deposit: November 15, 1988). A restriction site and function map of plasmid pOW390 is presented in Figure 1 of the accompanying drawings.

Plasmid pOW390 serves as useful starting material for the construction of other expression vectors of the invention. These vectors are especially useful in a method for producing racemase activity in a recombinant host cell, said method comprising: (1) transforming said host cell with a recombinant DNA expression vector that comprises: (a) a promoter and translational activating sequence; and (b) a DNA sequence that encodes racemase activity and is positioned for expression from said promoter and translational activating sequence; and (2) culturing said host cell transformed in step (1) under conditions that allow for expression of said racemase activity.

Plasmid pOW390 can be isolated from E. coli K12 RR1ΔM15/pOW390 by the procedure described in Example 1. Plasmid pOW390 contains the intact Streptomyces clavuligerus racemase gene, which can be isolated, for example, from the plasmid on an ~3.6 kb KpnI restriction fragment. Plasmid pOW390 was used as starting material in the construction of a plasmid, designated pOW392, that drives high-level expression of racemase activity in E. coli. To facilitate manipulation of the S. clavuligerus racemase coding sequence, a NcoI restriction enzyme recognition sequence was created at position -2 to +4 of the S. clavuligerus racemase DNA coding sequence in plasmid pOW392.

The creation of the NcoI site was done by M13 site-directed mutagenesis techniques and involved changing DNA bases surrounding the start codon of the racemase gene from ACATGG to CCATGG. One skilled in the art will recognize that DNA mutagenesis techniques are commonly used to introduce restriction enzyme recognition sites into DNA sequences and that in this case the encoded amino acid sequence was not changed. The mutagenesis and intermediate constructs are described in greater detail in Example 2.

Plasmid pOW392 can be constructed by inserting the Streptomyces clavuligerus racemase coding sequence into plasmid pCZR111 (available from the NRRL under accession number NRRL B-18249, date of deposit: August 11, 1987), an expression vector that comprises the lambda pL (λpL) promoter and a translation activating sequence, the cI857 temperature sensitive repressor gene, a tetracycline resistance-conferring gene, and DNA sequences encoding vector replication functions. For a detailed description of the construction of pOW392, see Example 2. The λpL-derived promoter of plasmid pCZR111 is positioned to drive expression of the racemase gene. At low temperatures of about 30°C, the cI857 protein encoded on plasmid pCZR111 or its derivatives is active and able to repress activity of the λpL promoter, but when the temperature is raised to about 42°C, the cI857 protein is inactivated, and the λpL promoter drives transcription of large amounts of mRNA encoding the gene product of interest. A restriction site and function map of plasmid pCZR111 is presented in Figure 2 of the accompanying drawings.

Plasmid pOW392 comprises the same cI857 gene, λpL promoter, and translational activating sequence as does plasmid pCZR111 but further contains the coding sequence of the racemase gene from plasmid pOW390 positioned for expression from the λpL promoter. The ~1.7 kb StyI restriction fragment of plasmid pOW390 comprises the complete coding sequence for racemase. Plasmid pOW392 was constructed so that the λpL promoter and the translational activating sequence of plasmid pCZR111 are positioned to drive expression of the racemase-activity-encoding DNA. A restriction site and function map of plasmid pOW392 is presented in Figure 3 of the accompanying drawings.

At temperatures of about 42°C, E. coli K12 JM109/pOW392 expresses racemase activity at high levels, approaching ~5% of the total cell protein. Crude cell extracts from these E. coli K12 JM109/pOW392 transformants are able to catalyze the conversion of isopenicillin N into penicillin N, whereas cell extracts from non-transformed E. coli K12 JM109 cells cannot catalyze this conversion. The method of assay and results of the assay for the conversion reaction are presented in Example 4.

Many E. coli K12 strains contain an endogenous penicillinase activity, probably encoded by the ampC locus. For this reason it is desirable to effect a partial purification of the racemase polypeptide so that optimal racemase activity is observed. For these purposes, purification of the enzyme can be used to separate the endogenous E. coli penicillinase activity from the desired racemase activity.

Plasmid pOW392 provides an efficient means of producing large amounts of racemase activity in E. coli. Because culturing E. coli is less complex than culturing organisms that naturally produce racemase, E. coli/pOW392 transformants can be used to produce recombinant racemase more efficiently and economically than non-recombinant or "natural" racemase producers.

Racemase can be used to produce penicillin N from isopenicillin N in a cell-free system as described in Example 3. Penicillin N is not only a useful antibiotic, but also can be used as the starting material for the production of such important antibiotics as cephalexin and other cephalosporins (see U.S. Patent No. 4,307,192). Perhaps the most important use of racemase is the use of the enzyme to transform penicillins

other than isopenicillin N into novel antibiotic derivatives. An example would be to use cephamycin C as a substrate for racemase, catalyzing the reaction in a direction reverse from its mode in natural biosynthetic pathways, to generate an L-aminoadipoyl derivative which could then serve as a substrate to form 7-aminocephalosporanic acid, a starting material for semi-synthetic cephalosporins.

Cell-free extracts of penicillin and cephalosporin-producing organisms can be used to synthesize unnatural (not produced in nature) β-lactams. The E. coli expression vectors of the present invention provide an inexpensive and efficient method of obtaining racemase activity, which can be used in vitro to modify penicillins to form novel antibiotics or antibiotic core structures.

Plasmid pOW392 is especially preferred for driving expression of racemase activity in E. coli not only because of the high expression levels achieved when using the plasmid but also because of the selectable marker present on the plasmid. Many recombinant DNA vectors encode a β-lactamase, so that cells containing the vector can grow in the presence of certain β-lactam antibiotics, such as ampicillin. However, if one desires to use a cell-free extract containing racemase activity for purposes of constructing β-lactams, one does not want the extract to contain β-lactamase activity. Thus, plasmid pOW392 does not encode a β-lactamase for a selectable marker but rather employs a tetracycline resistance-conferring gene, which encodes a protein that does not react with β-lactams.

The racemase expression vectors of the present invention, however, are not limited to a particular selectable marker. Those skilled in the art recognize that many selectable markers are suitable for use on racemase expression vectors. Such selectable markers include, for example, genes that confer kanamycin resistance, genes that confer chloramphenicol resistance, or other antibiotic resistance-conferring genes.

The search for unnatural penicillins that will serve as substrates for racemase can be complemented by a search for mutant racemase enzymes that will accept other penicillins as substrate. The present invention provides the starting material for such a search for a mutant racemase and comprises DNA compounds derived through mutagenesis of the racemase coding sequence. E. coli is a preferred host for mutational cloning experiments, and the E. coli expression vectors of the present invention can be readily mutated by procedures well known in the art, such as, for example, treatment with radiation (X-ray or UV) or chemical mutagens (such as ethylmethanesulfonate, nitrosoguanidine, or methyl methanesulfonate) or site-specific mutagenesis, to obtain mutant enzymes that recognize unnatural penicillins as substrate.

As those skilled in the art will recognize, the present invention allows one to change the codons for the racemase gene at will. Given the DNA sequence for the racemase gene, procedures familiar to one skilled in the art are used to generate mutant racemase enzymes that vary from the natural racemase enzyme at any number of amino-acid residue positions. Such mutant enzymes would be encoded by mutant racemase coding sequences, including sequences in which amino-acid codons have been deleted from or inserted into the natural racemase coding sequence. Such mutant enzymes are within the scope of the present invention, because even if one cannot absolutely predict whether a given mutation will destroy activity of the encoded racemase, one need merely express the mutant sequence to ascertain the effect on racemase activity.

The present invention is not limited to the particular vectors exemplified herein. Instead, the present invention comprises DNA compounds that encode the racemase activity of Streptomyces clavuligerus. The DNA compounds of the present invention can be used to construct expression vectors that drive expression of racemase activity in any host cell in which the expression vector replicates or integrates and in which the promoter and translational activating sequence are functional.

Therefore, the present invention comprises any E. coli expression plasmid or vector that drives expression of racemase activity in E. coli. Thus, the present invention comprises expression vectors that drive expression of racemase activity and utilize a replicon functional in E. coli, such as, for example, a replicon from such plasmids as pBR322, pACYC184, F, ColV-K94, R1, R6-5, or R100. Nor is the present invention solely limited to plasmid vectors, for the present invention also comprises expression vectors that express racemase activity and utilize integration or viral replication to provide for replication and maintenance in the host cell.

The present invention is not limited to a particular promoter and translational activating sequence to drive expression of the racemase activity-encoding DNA. The present invention comprises the use of any promoter and translational activating sequence that function in E. coli and is used to express racemase activity in E. coli. Many promoter and translational activating sequences functional in E. coli are known and are suitable for driving expression of racemase activity in E. coli. Such transcriptional and translational activating sequences include, but are not limited to, the lpp, lac, trp, tac, λpL, and λpR promoter and translational activating sequences.

In addition, transcriptional and translational activating sequences from other organisms can be ligated to the present racemase-activity-encoding DNA compounds to form expression vectors that drive expression

of isopenicillin N epimerase activity in host cells in which the activating sequence functions. Although E. coli is the host best suited for racemase production and subsequent purification for in vitro use, vectors that drive expression of isopenicillin N epimerase activity in host cells other than E. coli are also useful, especially for purposes of increasing the antibiotic-producing ability and efficiency of a given organism.

A variety of organisms produce β-lactam antibiotics. The following Table presents a non-comprehensive list of β-lactam antibiotic-producing organisms.

TABLE I

β-Lactam Antibiotic-Producing Organisms

| Organism | Antibiotic |
|---|---|
| Agrobacterium | various β-lactams |
| Arachnomyces minimus | penicillins and cephalosporins |
| Anixiopsis peruviana | penicillins and cephalosporins |
| Cephalosporium acremonium, purpurascens, polyaleurum, chrysogenum, curtipes | penicillins and cephalosporins |
| Chromobacterium | various β-lactams |
| Emericellopsis terricola, minima, synnematicola, glabra, mirabilis, salmosynnemata | penicillins and cephalosporins |
| Flavobacterium | various β-lactams |
| Gluconobacter | various β-lactams |
| Nocardia lactamadurans | cephamycin C |
| Nocardia uniformis | nocardicin |
| Paecilomyces carneus, persicinus | penicillins and cephalosporins |

| | |
|---|---|
| *Penicillium chrysogenum* | various penicillins and other β-lactams |
| *Serratia* | various β-lactams |
| *Spiroidium fuscum* | penicillins and cephalosporins |
| *Streptomyces* | |
| *antibioticus* | clavulanic acid |
| *argenteolus* | asparenomycin A, MM 4550, and MM 13902 |
| *cattleya* | thienamycin |
| *chartreusis* | SF 1623 and cephamycin A and B |
| *cinnamonensis* | cephamycin A and B |
| *clavuligerus* | PA-32413-I, cephamycin C, A16886A, penicillins, cephalosporins, clavulanic acid, and other clavams |
| *fimbriatus* | cephamycin A and B |
| *flavovirens* | MM 4550 and MM 13902 |
| *flavus* | MM 4550 and MM 13902 |
| *fulvoviridis* | MM 4550 and MM 13902 |
| *griseus* | cephamycin A and B and carpetimycin A and B |
| *halstedi* | cephamycin A and B |
| *heteromorphus* | C2081X and cephamycin A and B |
| *hygroscopicus* | deacetoxy-cephalosporin C |
| *lipmanii* | cephamycin, penicillin N, 7-methoxycephalosporin C, A16884, MM4550, MM13902 |
| *olivaceus* | epithienamycin F, MM 4550, and MM 13902 |
| *panayensis* | C2081X and cephamycin A and B |
| *pluracidomyceticus* | pluracidomycin A |
| *rochei* | cephamycin A and B |
| *sioyaensis* | MM 4550 and MM 13902 |
| sp. OA-6129 | OA-6129A |
| sp. KC-6643 | carpetimycin A |
| *tokunomensis* | asparenomycin A |
| *viridochromogenes* | cephamycin A and B |
| *wadayamensis* | WS-3442-D |

Some of the foregoing β-lactam antibiotic-producing organisms are used in the pharmaceutical industry for purposes of antibiotic production. The antibiotic-producing ability of these organisms can be increased and made more efficient by increasing the intracellular concentration of the antibiotic biosynthetic enzymes during the fermentation. The racemase activity-encoding DNA compounds of the present invention can be used to construct expression vectors that, when transformed into the appropriate host cell, increase the intracellular concentration of racemase activity of the transformed host cell and thereby increase the antibiotic-producing ability and efficiency of that cell, provided that the host cell produces a β-lactam antibiotic via an intermediate reaction involving racemase activity.

A vector that will increase the intracellular concentration of racemase activity of a given host cell into which the vector is transformed requires the following elements: 1) a racemase activity-encoding DNA compound of the present invention; and 2) a promoter and translational activating sequence that not only function in the host cell to be transformed, but also are positioned in the correct orientation and position to drive expression of the racemase activity-encoding DNA. Of course, stable transformants can only be obtained if the vector replicates, either as an extrachromosomal element or integrated in the genomic DNA, in the host cell. Thus, a preferred vector might contain sequences that specifically direct replication or integration of the vector in the host cell. However, the presence of such specific replication or integration sequences is not absolutely required, as non-specific integration may occur when DNA is introduced into a host cell. A racemase expression vector could also comprise an antibiotic resistance-conferring gene or some other element that provides a means of selecting for host cells which contain the vector, but such selectable elements may neither be necessary nor desired when the vector integrates into the chromosomal DNA of the host cell.

By providing the coding sequence of the racemase gene of Streptomyces clavuligerus, the present invention provides racemase expression vectors for any organism susceptible to transformation. The E. coli racemase expression vectors described above illustrate the wide variety of expression vectors of the present invention. However, many of the preferred vectors of the invention are designed to drive expression of racemase in a β-lactam antibiotic (including penicillins and cephalosporins) producing cell.

The Penicillium vectors of the invention are illustrative of the vectors provided by the present invention that can be used to increase the yield of antibiotic from such a β-lactam antibiotic-producing cell or to alter the antibiotic normally produced by the cell. One such illustrative vector, designated plasmid pPSJ78, contains the promoter and translational activating sequence of the Penicillium isopenicillin N synthetase (IPNS) gene positioned to drive expression of the racemase coding sequence of the present invention. A restriction site and function map of plasmid pPSJ78 is provided in Figure 5 of the accompanying drawings.

The construction protocol for plasmid pPSJ78 and a variety of useful intermediates is set forth in Example 4. The first step in constructing pPSJ78 is to digest plasmid pOW392 with restriction enzyme EcoRI which cuts the plasmid one time. The resulting linear DNA is then partially digested with restriction enzyme NcoI such that the ~500 bp EcoRI-NcoI fragment containing the natural Streptomyces clavuligerus promoter and translational activating sequence is removed. In its place is inserted the ~925 bp EcoRI-NcoI fragment from plasmid pLC2 (available from the American Type Culture Collection as ATCC 53334, date of deposit: November 20, 1985) which contains the Penicillium chrysogenum isopenicillin N synthetase promoter and translational activating sequence. The resultant plasmid is entitled pPSJ77 (Figure 7). The final step in constructing plasmid pPSJ78 is to insert an ~5.65 kb EcoRI fragment containing the Aspergillus nidulans acetamidase gene from plasmid pPS51 into EcoRI-digested pPSJ77. The acetamidase gene functions as a selective marker in Penicillium.

Racemase expression vectors that contain the acetamidase gene are particularly useful as vectors for inserting genes into Penicillium chrysogenum, because no special recipient strain, such as an auxotroph, need be constructed, owing to the natural inability of P. chrysogenum to grow on acetamide as sole nitrogen source. Transformation systems based on complementation of auxotrophic markers by a gene in the transforming plasmid do not share this advantage. Frequently, pleiotropic mutations are associated with the introduction of an auxotrophic marker into a P. chrysogenum strain highly developed for penicillin production. Such mutations usually result in lower penicillin production (MacDonald et al., 1963, J. Gen. Microbiol. 33: 365-374).

The vectors described above and in the Examples are merely illustrative of the wide variety of racemase expression vectors provided by the present invention. European Patent Publication Serial No. 0281391, published September 7, 1988, describes the 5′ and 3′ regulatory signals of the Cephalosporium acremonium expandase-hydroxylase gene. The signals can be combined with the racemase coding sequence of the present invention to yield racemase expression vectors of the invention especially suited for use in Cephalosporium. European Patent Publication 0200425, published December 10, 1986, discloses the transcription and translation activating sequences of the Cephalosporium acremonium IPNS gene, which can be fused to the Streptomyces clavuligerus racemase coding sequence of the present invention to create a recombinant racemase gene that drives expression (when incorporated into an expression vector and the vector introduced into Cephalosporium) of the S. clavuligerus racemase coding sequence in Cephalosporium.

The racemase expression vectors of the present invention are useful for increasing the intracellular concentration of racemase activity in any cell, especially β-lactam antibiotic-producing cells. Plasmid pOW390 comprises the coding sequence of the racemase gene of Streptomyces clavuligerus, so plasmid pOW390 can be used to construct vectors for increasing the copy number of the racemase gene and thus

for increasing intracellular concentration of the enzyme. Because the racemase coding sequence of the invention was isolated from a Streptomyces host cell, the racemase coding sequence is particularly well-suited for use in expression vectors designed to drive high-level expression of racemase activity in Streptomyces host cells. The literature is replete with techniques for constructing Streptomyces expression vectors and for transforming Streptomyces host cells. See, for instance, Garcia-Dominguez et al., 1987, Applied and Environmental Microbiology 53(6):1376-1381. The racemase coding sequence of the invention can be readily incorporated into an expression vector that comprises a Streptomyces promoter and replicon. A variety of known Streptomyces promoters and replicons are available for such use. Table II is an illustrative, but not comprehensive, listing of Streptomyces plasmids from which Streptomyces replicons can be obtained. Those skilled in the art recognize that, so long as the replicon function is not disrupted, all or part of the plasmids listed in the Table may be used to construct vectors that contain the racemase gene of the present invention. The plasmid-containing host and depository accession number are also listed in Table II.

TABLE II

| Streptomyces Plasmids | | |
|---|---|---|
| Plasmid | Host | Accession Number |
| SCP2 | Streptomyces coelicolor A3(2) | NRRL 15042 |
| SCP2* | Streptomyces coelicolor M110 | NRRL 15041 |
| pEL7 | Streptomyces ambofaciens/pEL7 | NRRL 12523 |
| pUC6 | Streptomyces espinosus | NRRL 11439 |
| pUC3 | Streptomyces 3022A | NRRL 11441 |
| SLP1 | Streptomyces lividans | NCIB[1] 11417 |
| pNM100 | Streptomyces virginiae | NRRL 15156 |
| pEL103 | Streptomyces granuloruber A399 12.13/pEL103 | NRRL 12549 |
| pIJ702 | Streptomyces lividans | ATCC[2] 39155 |

[1]National Collection of Industrial Bacteria (NCIB), Torry Research Station, Post Office Box 31, 135 Abbey Road, Aberdeen AB98DG, Scotland, United Kingdom.
[2]American Type Culture Collection, Rockville, MD 20852.

The Streptomyces clavuligerus racemase coding sequence of the invention can also be put under the control of transcription and translation activating sequences derived from other strains of Streptomyces, as well as from Penicillium, Cephalosporium, or any other host cell to construct a recombinant racemase gene for use in the given organism.

The following Examples are provided to further illustrate and exemplify, but do not limit the scope of, the present invention.

Example 1

## A. Culture of E. coli K12 RR1ΔM15/pOW390

A lyophil of E. coli K12 RR1ΔM15/pOW390 can be obtained from the Northern Regional Research Laboratories (NRRL), Peoria, IL 61604, under the accession number NRRL B-18431 (date of deposit: November 15, 1988) and used directly as the "culture" in the process described below.

One liter of TY broth (8 g tryptone, 5 g NaCl, and 5 g yeast extract per liter) containing 100 μg/mL ampicillin was inoculated with a culture of E. coli K12 RR1ΔM15/pOW390 and incubated with aeration at 37°C overnight (15-18 hours). The resulting culture was used as a source of plasmid pOW390.

B. Isolation of Plasmid pOW390

The culture prepared in Example 1A was centrifuged at 5200 rpm for 10 minutes at 4°C to pellet the cells. The resulting supernatant was discarded. The cell pellet was resuspended in 28 mL of a solution of

25% sucrose and 50 mM EDTA. About 1 mL of a solution of 20 mg/mL lysozyme in 50% glycerol and 0.25 M Tris-HCl, pH = 8.0, and about 1.5 mL of 0.5 M EDTA, pH = 8.0, were added to and mixed with the cell suspension. The resulting mixture was incubated on ice for 15 minutes. Three mL of lysing solution (prepared by mixing 3 mL of 10% Triton X-100; 75 mL of 0.25 M EDTA; pH = 8.0; and 7 mL of water) were added to the lysozyme-treated cells with gentle mixing. The resulting solution was incubated on ice for another 15 minutes.

The cellular debris was removed from the solution by centrifugation at 17,000 rpm for about 45 minutes at 4 °C. About 28.6 g of CsCl and ~1 mL of a 5 mg/mL ethidium bromide solution were added to the ~30 mL of supernatant. Then, the volume was adjusted to 40 mL with water and the solution decanted into an ultracentrifuge tube. The tube was sealed, and the solution was centrifuged at 49,500 rpm for ~18 hours. The resulting plasmid band, visualized with ultraviolet light, was isolated, extracted with CsCl-saturated isopropanol to remove the ethidium bromide, and dialysed against three changes of ~20 volumes of TE buffer (10 mM Tris-HCl, pH = 7.5, and 1 mM EDTA). The dialysate was collected; then, two volumes of ethanol and 0.05 volumes of 3 M sodium acetate solution were added. The ethanol mixture was cooled to -20 °C, and the plasmid DNA was pelleted by centrifugation at 10,000 rpm for 30 minutes at -10 °C. The resulting pellet was resuspended in ~1 mL of TE buffer and then extracted with an equal volume of a phenol:chloroform mixture (1:1, v/v). The DNA in the aqueous phase was recovered by the addition of 0.1 volume of 3 M NaOAc and 2 volumes of ethanol, followed by incubation at -20 °C for ~30 minutes and centrifugation at 15,000 rpm for 20 minutes. The resulting DNA pellet was rinsed first with 70% ethanol and then with 100% ethanol and dried.

The ~1.5 mg of plasmid pOW390 DNA obtained by this procedure was suspended in 1.5 mL of 0.1X TE buffer and stored at -20 °C. A restriction site and function map of plasmid pOW390 is presented in Figure 1 of the accompanying drawings.

## Example 2

### Construction of Plasmid pOW392

#### A. Construction of Plasmid mOW390

Identical plasmid constructs can be achieved employing different methods and sources of gene sequences. Plasmid mOW390 was constructed by ligating the ~0.9 kb StyI-BamHI restriction fragment of plasmid pOW390, prepared as noted in B., below, with HincII-BamHI-digested, replicative form (RF) M13 vector, prepared as noted in C., below. The ~0.9 kb StyI-BamHI fragment contains a large portion of the racemase gene, including the 5′ end, but not the 3′ end. The StyI end was "filled in" so as to make it blunt-ended, rendering the end amenable to ligation with the blunt-ended HincII end. The plasmid pOW390 clone originated from cosmid pOW379, derived from a *Streptomyces clavuligerus* genomic cosmid library. Cosmid pOW379 was identified by hybridization using a "guessmer" DNA probe designed on the basis of the amino-terminal amino acid residue sequence of purified *S. clavuligerus* racemase and species codon-usage bias. The desired phage M13 clone can be identified using the "guessmer" probe in a plaque hybridization procedure or by restriction enzyme analysis. Because of the present invention, however, the construction of mOW390 is greatly simplified, primarily because plasmid pOW390 can be used as the source of the *S. clavuligerus* racemase gene. The M13-derived plasmid mOW390 was a useful intermediate in the site-specific mutagenesis carried out to create an NcoI restriction enzyme recognition site at the 5′ end of the *S. clavuligerus* racemase coding sequence.

#### B. Isolation of the ~0.9 kb BamHI-StyI Restriction Fragment from Plasmid pOW390

Approximately 25 µg of the plasmid pOW390 DNA in 25 µl 0.1X TE buffer, as prepared in Example 1B, were added to and mixed with 40 µl of 10X StyI buffer (1.0 M NaCl; 500 mM Tris-HCl, pH = 8.0; and 100 mM $MgCl_2$), 335 µl of glass-distilled water, and 5 µl (~50 units) of restriction enzyme StyI. Unless otherwise noted, restriction enzymes were obtained from New England Biolabs, 32 Tozer Road, Beverly, MA 01915. Unit definitions herein correspond to the particular manufacturer's unit definitions. The resulting reaction was incubated at 37 °C for 90 minutes. The StyI ends were then made blunt ("filled-in") by the method of Maniatis *et al.*, *Molecular Cloning: A Laboratory Manual*, Cold Spring Harbor Laboratory, 1982, pp. 113-114.

The reaction was then extracted with phenol and chloroform, and the StyI-digested plasmid pOW390 DNA was precipitated with 0.3 M NaOAc and ethanol and then resuspended in 45 µl of 1X BamHI buffer (100 mM NaCl; 10 mM Tris-HCl, pH = 7.5; 10 mM $MgCl_2$). Five µl (~50 units) of restriction enzyme BamHI were added and the mixture incubated at 37°C for 90 minutes. The reaction was then extracted with phenol and chloroform, and the BamHI-StyI (blunt)-digested plasmid pOW390 was precipitated with 0.3 M NaOAc and ethanol and then resuspended in 9 µl of $H_2O$. About 1 µl of loading buffer (25% v/v glycerol, 0.05% w/v bromphenol blue, and 0.05% xylene cyanol) was added to the solution of DNA, which was then electrophoresed on a 1% agarose gel until the desired ~0.9 kb BamHI-StyI restriction fragment was clearly separated from the other digestion products.

The electrophoresed DNA was visualized by staining the gel in a dilute solution (0.5 µg/ml) of ethidium bromide and exposing the stained gel to long-wave UV light. After the fragments were located, a small slit was made in the gel in front of the ~0.9 kb fragment, and a piece of Schleicher and Schuell (Keene, NH 03431) DEAE membrane was placed in the slit. Upon further electrophoresis, the DNA non-covalently bound to the DEAE membrane. After the desired fragment was bound to the DEAE membrane, the membrane was removed and rinsed with low salt buffer (100 mM NaCl; 0.1 mM EDTA; and 20 mM Tris-HCl, pH = 8). Next, the membrane was placed in a small tube and immersed in high salt buffer (1 M NaCl; 0.1 mM EDTA; and 20 mM Tris-HCl, pH = 8) and then incubated at 65°C for 10 minutes to remove the DNA from the DEAE paper. After the 65°C incubation, the incubation buffer was collected, and the membrane was rinsed with high salt buffer. The rinse solution was pooled with the incubation buffer before collecting the desired DNA fragments.

The volume of the high salt-DNA solution was adjusted so that the NaCl concentration was 0.25 M, and then three volumes of cold, absolute ethanol were added to the solution. The resulting solution was mixed and placed on ice for 10-20 minutes. The solution was then centrifuged at 15,000 rpm for 15 minutes. After another precipitation to remove residual salt, the DNA pellet was rinsed with 70% ethanol, dried, resuspended in 20 µl of TE buffer, and constituted the desired restriction fragment. About 0.2 µg of the ~0.9 kb fragment was obtained.

## C. Preparation of BamHI-HincII-Digested Vector M13mp18 RF DNA and Construction of Plasmid mOW390

About 2.5 µg of M13mp18 RF DNA (available from New England Biolabs (NEB)) were digested in 100 µl of BamHI buffer with 1 µl (~20 units) of restriction enzyme BamHI for 90 minutes at 37°C. The reaction mixture was extracted with phenol:chloroform and the DNA, in the aqueous phase, concentrated by ethanol precipitation.

The BamHI-digested M13mp18 was resuspended in 100 µl HincII buffer (10 mM Tris-HCl, pH = 7.4, 7 mM MgCl, and 1 mM DTT) with 1 µl (~10 units) of restriction enzyme HincII for 90 minutes at 37°C. The reaction was extracted and precipitated as above and resuspended in 20 µl 0.1 X TE buffer and constituted ~2 µg of the desired BamHI-HincII digested vector. The other BamHI-HincII fragment was only ~10 bp and was not precipitated. It did not interfere with the ligation.

Two µl of the ~0.9 kb BamHI-StyI restriction fragment of plasmid pOW390 from B., above, and 1 µl of BamHI-HincII-digested vector M13mp18 were ligated in a 20 µl reaction containing the DNA fragments, 2 µl of 10X ligase buffer (0.5 M Tris-HCl, pH 7.5, and 100 mM $MgCl_2$), 2 µl of 5 mM ATP, 1 µl of 6 µg/µl BSA, 12 µl of glass-distilled water, and 1 µl (1 Weiss unit) of T4 DNA ligase (NEB). The "filled-in" StyI ends were ligatable with the HincII blunt ends. The reaction was incubated ~18 hours at 15°C. The ligated DNA constituted the desired plasmid mOW390 along with other ligation products.

Competent E. coli K12 JM109 ("Epicurean Coli™") were purchased from Stratagene (3770 Tansy Street, San Diego, CA 92121) and transformed with a ligation reaction mixture constituting plasmid mOW390 in substantial accordance with the manufacturer's directions, except that the DNA was in a volume of 20 µl and no dilution into medium or expression time was necessary. Post-transformation, the cells were distributed in ~1, 10, 20, 40 and 50 µl samples to 13 X 100 mm sterile glass tubes containing 0.25 mL/tube E. coli K12 JM109 in logarithmic growth phase. To these tubes were added 3 mL of top agar (L broth with 0.8% agar kept molten at 45°C). The cell-top agar mixture was then plated on L-agar plates containing 40 µg/mL 5-bromo-4-chloro-3-indolyl-β-D-galactoside (X-gal) and 0.1 M isopropylthio-β-galactoside (IPTG), and the plates were incubated at 37°C overnight. (For more detailed descriptions and explanations of M13 procedures, see M13 Cloning/Dideoxy Sequencing Instruction Manual, Bethesda Research Laboratories (BRL), Life Technologies, Inc., Gaithersburg, MD 20877.) Transformants are identified by insertional inactivation of β-galactosidase activity (colorless plaque phenotype) and restriction enzyme analysis of replicative form (RF) DNA. For screening purposes, clear plaques are plugged from the plate overlay with a Pasteur pipette into 3 mL per plaque of early logarithmic growth phase E. coli K12 JM109. Cultures are incubated from 6 to 18 hours at 37 °C with aeration.

Following this incubation, 1.5 mL of each culture are pelleted in separate 1.5 mL Eppendorf tubes. The supernatants are decanted into fresh tubes and stored at 4 °C to serve as a source of phage inoculum. Replicative form DNA is prepared from the cell pellets in substantial accordance with the teaching of the alkaline plasmid preparation procedure of Birnboim and Doly, 1979, Nuc. Acid Res. 7(6): 1513-1523, with the following exceptions. The procedure is scaled up such that 1.5X volumes of Solutions I, II, and III are used, and the cleared lysate is extracted once with an equal volume of $CHCl_3$. The DNA is then precipitated by the addition of 0.4 volumes of isopropanol and incubation at room temperature for 20 minutes. The DNA is collected by centrifugation and then precipitated with ethanol out of 0.3 M NaOAc. The DNA isolated from individual plaques was analyzed for the presence of an insert by EcoRI-HindIII restriction enzyme digestion. The orientation of the fragment was pre-determined by the compatible ends (BamHI-BamHI, "filled-in" StyI-HincII). By this method, E. coli K12 JM109/mOW390 cells were identified; these cells were then used as a source of plasmid mOW390 for the site-specific mutagenesis, as described below.

D. Preparation of Single-Stranded Plasmid mOW390 DNA and Site-Specific Mutagenesis to Construct Plasmid mOW391

A 10 mL culture of early logarithmic growth phase E. coli K12 JM109 was inoculated with ~200 µl of phage stock (prepared in Example 2C) and incubated ~18 hours at 37 °C with aeration. The culture was centrifuged and the resulting supernatant transferred to a new tube and recentrifuged. The supernatant was again decanted to a fresh tube. One mL of a solution of 25% polyethylene glycol (molecular weight ≅ 3,350) in 3 M NaCl was added to the supernatant, which was then incubated for 15 minutes at room temperature. The resulting mixture was centrifuged for 30 minutes at 10,000 rpm. The pellet obtained by the centrifugation contained the single-stranded plasmid mOW390 and was resuspended in 400 µl of TE buffer. The solution was extracted first with $CHCl_3$ and then with TE-saturated phenol. The phenol was allowed to stay in contact with the aqueous phase for 15 minutes. The solution was then extracted twice with a mixture of TE-saturated phenol:$CHCl_3$ (1:1, v/v), and twice with $CHCl_3$ alone. The DNA was then precipitated out of 0.3 M NaOAc, collected by centrifugation, and the resulting pellet resuspended in 100 µl of 0.1X TE buffer. This solution constituted ~5 µg of single-stranded plasmid mOW390 DNA.

E. Mutagenesis

The single-stranded DNA fragments used in the mutagenesis (and subsequent hybridizations to detect desired phages) were synthesized on an automated DNA synthesizer, with the exception of the M13 universal primer (a 15-mer), which was purchased from BRL. The mutagenesis fragments were designated as follows: (1) RACEATG, a single-stranded DNA 40 nucleotides in length that is homologous to the racemase coding sequence in plasmid mOW390 except for 1 base, the mismatch (underlined) of which will create a restriction enzyme NcoI recognition sequence at about position 1 of the racemase coding sequence, with the DNA sequence:

NcoI
5'-GCGGGAGATGCGTTTGCCATGGCGGTAGCCGACTGGGAAG-3'

(2) RACE-17, a single-stranded DNA 17 nucleotides in length that is merely a subfragment of RACEATG with the DNA sequence:

NcoI
5'-TGCGTTTGCCATGGCGG-3'

The 5′ ends of about 100 pmols of RACEATG were phosphorylated (kinased) in a reaction mixture containing single-stranded DNA at a concentration of 1 pmol/µl, 10 µl of 10X ligase buffer, 10 µl 0.1 mM adenosine triphosphate (ATP), 10 µl of 0.1 M DTT, 65 µl of glass-distilled water, and 1 µl (10 Richardson units) of T4 polynucleotide kinase (Boehringer-Mannheim Biochemicals, (BMB) 7941 Castleway Drive, P.O.

Box 50816, Indianapolis, Indiana 46250). The reaction mixture was incubated at 37°C for 30 minutes, at which time an additional 1 µl of enzyme was added. The reaction mixture was then incubated for another 30 minutes at 37°C and then quenched by incubation at 68°C for 5 minutes. The 5′ ends of about 40 pmols of M13 universal primer were kinased in an analogous 40 µl of reaction mixture containing the same amount of enzyme.

The single-stranded plasmid mOW390 DNA was mutagenized in substantial accordance with the teaching of Adelman *et al.*, 1983, DNA 2(3): 183-193 as described below. The annealing reaction was carried out by adding ~500 nanograms (in 15 µl of 0.1X TE buffer) of single-stranded plasmid mOW390 DNA to 8 µl of 10X annealing buffer (100 mM Tris-HCl, pH = 7.5; 1 mM EDTA; and 500 mM NaCl), 4 µl (4 pmols) of kinased RACEATG, 4 µl (4 pmols) of kinased M13 universal sequencing primer, and 50 µl of water, incubating the mixture at 80°C for 2 minutes, then at 55°C for 5 minutes, and finally at room temperature for 5 minutes.

The extension reaction was carried out by adding 120 µl of the following mixture to the solution of annealed DNA: 20 µl 10X Klenow-ligase buffer (100 mM Tris-HCl, pH = 7.5; 1 mM EDTA; and 500 mM NaCl), 20 µl of 0.1 M DTT; 20 µl of a solution 6.25 mM in each of dGTP, dATP, TTP, and dCTP; 20 µl of 5 mM ATP; 120 µl of water; 3 µl (3 Weiss units, BMB) ligase, and 2.5 µl (12.5 units) of Klenow enzyme (BMB). The extension reaction mixture was incubated at room temperature for 1 hour, then at 37°C for 4 hours, and finally at 14°C for ~18 hours.

The extension reaction mixture was extracted once with $CHCl_3$ and the DNA precipitated with ethanol and NaOAc and collected by centrifugation. The DNA pellet was resuspended in 400 µl 1X S1 buffer (0.3 M NaCl and 3 mM $Zn(OAc)_2$). Half the DNA solution was held in reserve at -20°C; half was aliquoted to five 1.5 mL tubes. To four of these tubes was added 1 µl of S1 nuclease (BMB) that had been diluted to 200 30-minute units per µl. The reactions were incubated at room temperature for 5, 10, 15, and 20 minutes, respectively. The reactions were stopped by first adding 5-10 µg of tRNA to the reaction mixture to serve as carrier, then extracting with a TE-saturated phenol-$CHCl_3$ mixture (1:1, v/v). The sample that was not treated with S1 (the negative control) was also extracted. The DNA in the aqueous phase was concentrated by ethanol precipitation and collected by centrifugation. The DNA pellets were each resuspended in 20 µl water.

Ten µl of each of the resulting S1-treated DNA solutions were used to transform *E. coli* K12 JM109 in substantial accordance with the procedure described in Example 2B, except that the plates did not contain either X-Gal or IPTG. Desired mutants were identified by hybridization of radiolabelled oligonucleotide RACE-17 to plasmid DNA blotted onto nitrocellulose filters as described below.

After plaque formation, the plates were incubated at 4°C for ~1 hour to harden the top agar. Nitrocellulose filters were placed on top of the lawn of each of two plates, containing ~50-200 plaques, from each of the negative control, the 10 minute S1-treated series, and the 20 minute S1-treated series. Contact between the filter and the surface of the lawn was maintained for ~1 minute, at which time the nitrocellulose filter was treated, by using saturated 3MMChr filter papers (Whatman Labsales, Inc., P.O. Box 1359, Hillsboro, Oregon 97123-1359), with 0.1 N NaOH-1.5 M NaCl for ~5 minutes, then 0.5 M Tris-HCl(pH = 7.0)-3 M NaCl for ~5 minutes. The nitrocellulose filters were air-dried and then baked *in vacuo* at 80°C for 30 minutes.

The nitrocellulose filters were prehybridized for ~5 minutes at room temperature in a solution of 6X SSC (20X SSC is 3 M NaCl and 0.3 M sodium citrate), 10X Denhardt's solution (0.2 g of polyvinylpyrollidone), 0.2 g of bovine serum albumin, and 0.2 g of Ficoll per 100 mL of water), 0.1% NaPPi, 0.1% SDS, and 10 µg/mL of denatured *E. coli* chromosomal DNA. The filters were then hybridized in a solution of 6X SSC, 10X Denhardt's solution, 0.1% NaPPi, and 1 pmol/5 mL of $^{32}$P-RACE-17. The $^{32}$P-RACE-17 was prepared by phosphorylating the 5′ ends of 100 pmols of RACE-17 in substantial accordance with the procedure described earlier in this example, except that ~70 pmol of $\gamma$-$^{32}$P-ATP (New England Nuclear (NEN), 549 Albany Street, Boston, MA, 02118, Catalog # NEG-002A) were used instead of non-radioactive ATP. After hybridization, the filters were rinsed twice for 5 minutes per wash in excess 6X SSC at room temperature, then at 52°C in excess 6X SSC for 20 minutes per wash. The filters were air-dried and autoradiographed for 2 hours at -70°C with a Quanta III® intensifying screen (DuPont, Instrument Products, Biomedical Division, Newtown, CN 06470). Desired mutants, those containing sequences complementary to the sequence of RACE-17, exposed the film due to binding of the radiolabelled oligomer by the plasmid DNA bound to the filter. The identity of a correct mutant, designated plasmid mOW391, was confirmed by restriction analysis of its RF DNA, which was prepared in substantial accordance with the procedure described in Example 2C.

F. Final Construction of Plasmid pOW392

The RF DNA of plasmid mOW391 contains the racemase coding sequence on the NcoI-BamHI restriction fragment utilized in the construction of the E. coli expression plasmid pOW392.

Replicative form DNA from plasmid mOW391-infected E. coli K12 JM109 was isolated in substantial accordance with the procedure described in Example 2C. About 10 µg of the RF DNA of plasmid mOW391 DNA were digested with restriction enzyme BamHI (~10 units) and NcoI (~10 units) in a reaction containing the DNA in 1X React®3 buffer (500 mM Tris-HCl, pH = 8.0, 100 mM $MgCl_2$, 1 M NaCl; Bethesda Research Laboratories, P.O. Box 6009, Gaithersburg, MD 20877). After incubation for ~90 minutes at 37°C, the reaction mixture was subjected to agarose gel electrophoresis, and the ~0.9 kb BamHI-NcoI fragment was isolated in substantial accordance with Example 2B.

Plasmid pCZR111 is available in E. coli K12 JM109 from the NRRL under accession number B-18249 (date of deposit: August 11, 1987). It can be isolated in substantial accordance with Example 1 except that 15 µg/ml tetracycline is included in the culture medium rather than 100 µg/ml ampicillin. A restriction site and function map of pCZR111 is presented in Figure 2.

The vector was generated by digesting plasmid pCZR111 with XbaI and BamHI. About 1 µl of XbaI (~10 units) is added to 10 µl plasmid pCZR111 (~10 µg) and 5 µl 10X XbaI buffer (500 mM Tris-HCl, pH = 8.0, 100 mM $MgCl_2$, and 500 mM NaCl). After incubation at 37°C for 90 minutes, 0.5 µl of 5 M NaCl and 1 µl BamHI (~10 units) is added and the incubation continued at 37°C for an additional 90 minutes. The reaction mixture is then subjected to agarose gel electrophoresis, and the ~5.75 kb XbaI-BamHI vector fragment is isolated in substantial accordance with Example 2B.

An intermediate plasmid can be created by ligating the ~0.9 kb BamHI-NcoI fragment from mOW391, the ~5.75 kb XbaI-BamHI vector fragment from pCZR111 and a double-stranded XbaI-NcoI restriction fragment synthesized by the phosphotriester method to yield the ~6.7 kb intermediate plasmid. The double stranded DNA fragment has the following sequence:

```
5'-CTAGAGGGTATTAATAATGTATATTGATTTTAATAAGGAGGAATAATCC-3'
       ||||||||||||||||||||||||||||||||||||||||||||
       3'-TCCCATAATTATTACATATAACTAAAATTATTCCTCCTTATTAGGGTAC-5'
```

The ligation mixture is transformed into E. coli K12 JM109 in substantial accordance with Example 2C, and plasmid DNA isolated from transformants is analyzed by restriction enzyme digestion to confirm the presence of the correct inserts.

To complete the expression vector, an ~2 kb BamHI fragment was isolated from plasmid pOW390 in substantial accordance with Example 2B. This ~2 kb BamHI fragment comprises the 3′ coding region of the racemase gene. The completed vector, therefore, contains the entire coding region of the racemase gene.

The ~2 kb BamHI fragment (2.5 µl, ~0.5 µg) was ligated to the intermediate vector plasmid digested with BamHI in substantial accordance with the procedure described in Example 2C to form desired plasmid pOW392. A restriction site and function map is provided in Figure 3 of the drawings.

The ligation reaction constituting the desired plasmid pOW392 was transformed into competent E. coli K12 RR1ΔM15 (NRRL B-15440, date of deposit: May 27, 1983). Aliquots of the transformation mixture were plated on L-agar plates containing tetracycline (15 µg/mL). The plates were incubated at 37°C for ~18 hours. Tetracycline-resistant transformants were further screened by restriction enzyme analysis of their plasmid DNA to identify the desired plasmid pOW392 transformants. Plasmid DNA was prepared from 3 mL cultures in substantial accordance with the Birnboim and Doly procedure described above for preparing RF DNA from phage M13-infected E. coli K12 JM109 cell pellets. Plasmid pOW392 DNA from one transformant was prepared in substantial accordance with the procedure described in Example 1 for use in subsequent constructions. The plasmid was then transformed into E. coli K12 JM109 purchased from Strategene.

Example 3

## Assay of E. coli-produced Racemase Activity

### A. Culture of E. coli K12 JM109/pOW392 for Expression of Racemase Activity

An E. coli K12 JM109/pOW392 transformant was grown at 30°C overnight in 500 ml of L broth (containing 15 μg/ml of tetracycline) in a gyratory incubator. The cells were diluted 1:10 by adding 100 ml of the overnight culture to 900 ml of fresh medium containing 15 μg/ml tetracycline in a 2.8 L Fernbach flask and incubated a further hour at 30°C under the same growth conditions. The temperature of the air shaker was then raised to 42°C and incubation continued for an additional 6.5 hours. The cI857 temperature-sensitive repressor of the lambda pL promoter, positioned to drive racemase expression on plasmid pOW392, is inactivated at 42°C and so allows for expression of racemase. After induction, the cells were harvested by centrifugation and used as a preferred source of E. coli-produced racemase activity.

### B. Demonstration of Isopenicillin N Epimerase (Racemase) Activity in the E. coli K12 JM109/pOW392 Cells Grown at 42°C

Five grams of the induced, pelleted E. coli were mixed with 10 ml of Breaking Buffer (10 mM sodium pyrophosphate-HCl, pH = 8.0 in 5 M urea, 20% glycerol, and 0.1 mM dithiothreitol (DTT)). If the amount of cells after centrifugation was less than 5 gm, the ratio of cells to buffer was maintained.

The cell suspension was then cooled to 2°C in an ice-ethanol bath and was sonicated at full power for 20 seconds. The cooling and sonicating was repeated three times.

Cell debris was removed by centrifugation at 47,000 x g for 20 minutes at 4°C. The supernatant was then filtered through glass wool with the filtrate being the crude enzyme.

The enzyme catalyzes the conversion of isopenicillin N to penicillin N and vice-versa, creating an equilibrium state between the two molecules. Therefore, the activity was measured in two ways, once using isopenicillin N as the initial substrate, and once using penicillin N as the starting material. Total protein was measured by the method of Lowry et al., J. Biol. Chem. 193, 265 (1951). Cell extract was mixed with 1.4 mM isopenicillin N or penicillin N, 0.2 mM dithiothreitol, 0.1 mM pyridoxal 5′-phosphate (as measured by the hydrazine method (Wada, H. and Snell, E., J. Biol. Chem. 236, 2089 (1961)), 50 mM pyrophosphate-HCl, pH = 8.3 in a final volume of 0.5 ml. The reaction mixture was then incubated at 37°C for 20 minutes. The reaction was terminated by boiling for 10 minutes. It was then put through a 0.45 μm filter.

Penicillin N and isopenicillin N are difficult to separate by HPLC. The compounds must be derivatized with o-phthaldialdehyde according to the method of Aswad, D.W., Anal. Biochem. 137, 405 (1984) and Usher, J., Lewis, M., and Hughes, D.W., Anal. Biochem. 149, 105 (1985). Twenty μl of the filtrate were mixed with 5 μl of the derivatizing solution (4 mg o-phthaldialdehyde (Sigma Chemical Co., P.O. Box 14508, St. Louis, MO 63178) dissolved in 300 μl methanol, 250 μl 0.4 M sodium borate, pH = 9.4, 390 μl $H_2O$ and 60 μl 1 M N-acetyl-L-cysteine, adjusted to pH 5-6 with NaOH). The reaction was allowed to proceed for 2 minutes at room temperature before termination with 200 μl 50 mM sodium acetate, pH = 5.0. Fifty μl aliquots were analyzed by HPLC using a flow rate of 1 ml/min and measurement of fluorescence at 360 nm. When isopenicillin N was included in the mixtures the cell extracts expressing the racemase activity converted a portion of isopenicillin N to penicillin N. Likewise, penicillin N was converted to isopenicillin N in the presence of these cell extracts. No conversion was seen when the cell extract was not added to the mix. In addition, antibody to the purified racemase enzyme reacted with an ~50,000 dalton band on a Western blot. Taken together, these results indicate that the gene cloned results in a protein which has racemase activity and is the same size as the purified racemase enzyme.

Example 4

Construction of Plasmid pPSJ78

This example sets forth a construction protocol for plasmid pPSJ78, a racemase expression vector of the invention that contains the promoter of the Penicillium IPNS gene positioned to drive expression of the racemase coding sequence of the invention. The promoter of the Penicillium IPNS gene can be isolated from plasmid pLC2, available from the American Type Culture Collection (Rockville, MD 20852) under accession number ATCC 53334 (date of deposit: November 20, 1985). A restriction site and function map of plasmid PLC2 is presented in Figure 6 of the accompanying drawings.

A. Construction of Intermediate Plasmid pPSJ77

Plasmid pOW392 (as constructed in Example 2) is isolated in substantial accordance with Example 1 except that the growth medium contains 15 µg/ml tetracycline instead of 100 µg/ml ampicillin. Approximately 25 µg of the plasmid pOW392 DNA in 25 µl 0.1 X TE buffer is added to and mixed with 10 µl of 10 X REact™3 buffer (BRL), 60 µl of $H_2O$ and 5 µl (~50 units) of restriction enzyme EcoRI. The resulting reaction is incubated at 37°C for 90 minutes. About 10 µl (~100 units) of restriction enzyme NcoI is then added to the mixture and the reaction is further incubated at 37°C for 3 minutes. The reaction is then extracted once with phenol, once with phenol-chloroform (1:1 phenol:chloroform; chloroform is actually 23:1 chloroform:isoamyl alcohol), and once with chloroform. The EcoRI-digested NcoI-partially digested plasmid pOW392 is precipitated with 1/10th volume 2.5 M NaOAc (pH = 5.2) and 2 volumes ethanol. The mixture is chilled to -20°C for 20 minutes, then pelleted in an Eppendorf Centrifuge for 10 minutes. The DNA is resuspended in 18 µl of $H_2O$. About 2 µl of loading buffer (25% v/v glycerol, 0.05% w/v bromphenol blue, and 0.05% xylene cyanol) is added to the solution of DNA, which is then electrophoresed on a 1% agarose gel until the desired ~7.9 kb EcoRI-NcoI restriction fragment is clearly separated from the other digestion products.

The ~7.9 kb band is visualized and isolated from the gel in substantial accordance with Example 2B.

Plasmid pLC2, available from the ATCC under accession number 53334 (date of deposit: November 20, 1985), is isolated in substantial accordance with Example 1. The plasmid is also digested with EcoRI and NcoI. An ~925 bp EcoRI-NcoI restriction fragment is isolated from among the resulting digestion products. The ~925 bp EcoRI-NcoI fragment comprises the IPNS promoter from Penicillium. When this fragment is ligated to the ~7.9 kb EcoRI-NcoI fragment from plasmid pOW392 in substantial accordance with Example 2C, the resulting plasmid pPSJ77 comprises the Penicillium IPNS promoter positioned to drive expression of the racemase gene.

B. Construction of Intermediate Plasmid pPS51

About 15 µg of plasmid pMLC12 DNA (NRRL B-18097 (date of deposit: August 8, 1986) and Figure 9) were dissolved in 5 µl of 10X EcoRI buffer and 40 µl of water. The plasmid can be isolated in accordance with Example 1 except that the growth medium contains 25 µg/ml chloramphenicol instead of 100 µg/ml ampicillin. About 5 µl (~50 units) of restriction enzyme EcoRI were added to the solution of DNA, and the resulting reaction was incubated at 37°C for 3 minutes to produce a partial digestion. The reaction was terminated by extraction with buffered phenol, which was followed by extraction with chloroform. Plasmid pMLC12 contains two EcoRI restriction sites, the E. coli lacZα fragment and a chloramphenicol resistance gene (CAT). The desired partial cleavage of the EcoRI site was to occur within the lacZα fragment and not within the CAT gene. This EcoRI digestion produced a mixture of plasmid pMLC12 DNA molecules: uncut; cut at the undesired location; cut at the desired location; and cut at both locations, producing fragments smaller than the full-length ~2.7 kb molecules. The EcoRI-digested plasmid pMLC12 DNA was precipitated, collected by centrifugation, dissolved in 50 µl of TE buffer, and loaded onto a 0.8% preparative agarose gel. The full length linear molecules (i.e., ~2.7 kb) were isolated.

The partially EcoRI-digested plasmid pMLC12 DNA was dissolved in 5 µl of 10X SalI buffer and 40 µl of water. About 5 µl (~50 units) of restriction enzyme SalI were added to the EcoRI-linearized plasmid pMLC12 DNA, and the resulting reaction was incubated at 37°C for two hours. The unique SalI restriction site in plasmid pMLC12 is located 24 base pairs from the EcoRI site within the lacZα fragment of plasmid pMLC12. Thus, complete SalI digestion of the partially EcoRI-digested plasmid pMLC12 DNA produced four DNA fragments: one ~2.7 kb, the desired molecule; one ~24 bp in length; one ~0.6 kb; and one ~1.9 kb. The DNA molecules were size-fractionated on a 0.8% agarose gel. The nearly full-length, ~2.7 kb linear

molecules were isolated.

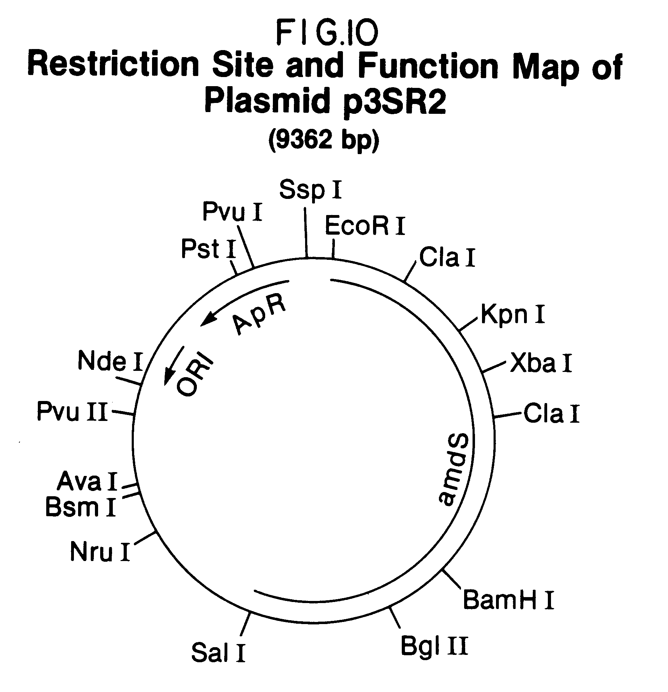

The acetamidase gene of Aspergillus nidulans can be isolated on an ~5.0 kb EcoRI-SalI restriction fragment of plasmid P3SR2 (Figure 10 and NRRL B-18182, date of deposit: February 26, 1987). About 10 μg of plasmid p3SR2 were dissolved in 5 μl of 10X EcoRI buffer and 40 μl of water. About 5 μl (~50 units) of restriction enzyme EcoRI were added to the solution of DNA, and the resulting reaction was incubated at 37°C for two hours. The reaction was terminated by extraction with buffered phenol, which was followed by extraction with chloroform. The EcoRI-digested p3SR2 plasmid DNA was precipitated, collected by centrifugation, and resuspended in 5 μl of 10X SalI buffer and 40 μl of water. About 5 μl (~50 units) of restriction enzyme SalI were added to the solution of DNA, and the resulting reaction was incubated at 37°C for two hours. The two DNA fragments generated in these digestions were size-fractionated on a 0.8% preparative agarose gel. One ~4.3 kb fragment comprised pBR322 DNA and the other ~5.0 kb fragment comprised the acetamidase (amdS) gene from Aspergillus nidulans. The amdS gene functions as a selectable marker in Penicillium and other strains. The gene enables growth on acetamide-containing media. The ~5.0 kb EcoRI-SalI fragment was isolated. About 3 μg of the ~5.0 kb EcoRI-SalI fragment were recovered and suspended in 5 μl of water.

One μl of the EcoRI-SalI-digested plasmid pMLC12 DNA was added to about 4 μl of the ~5.0 kb EcoRI-SalI restriction fragment of plasmid p3SR2, together with 2 μl of 10X ligase buffer, 2 μl of T4 DNA ligase, and 11 μl of water. The resulting ligation reaction was incubated at 15°C overnight. The ligated DNA constituted the desired plasmid pPS51 and other related ligation products.

This ligation mixture was used to transform E. coli K12 C600 (ATCC 33524). Aliquots of the transformed cell mixture were plated on L-agar plates containing 25 μg/ml of chloramphenicol. The plates were incubated at 37°C overnight. Colonies that contained a plasmid without an insert, such as E. coli K12 C600/pMLC12 were distinguished from colonies that contained a plasmid with an insert, such as E. coli K12 C600/pPS51, by restriction analysis. A colony was identified that contained a plasmid with an insert. Plasmid DNA from this colony was screened for the presence of the ~5.0 kb EcoRI-SalI restriction fragment containing the Aspergillus nidulans amdS gene and had the correct structure for the desired plasmid pPS51. A large scale plasmid preparation of plasmid pPS51 was made in substantial accordance with the teaching in Example 1. A restriction site and function map of plasmid pPS51 is presented in Figure 8 of the accompanying drawings.

C. Final Construction of Plasmid pPSJ78

The amdS gene is then isolated on an ~5.65 kb EcoRI fragment from pPS51 and inserted into pPSJ77 (from Example 4) to provide a selectable marker for use in Penicillium and other organisms. A transformant containing plasmid pPS51 is isolated in substantial accordance with Example 1 except that 25 μg/ml chloramphenicol is included in the growth medium rather than 100 μg/ml ampicillin. About 25 μg of plasmid pPS51 is digested with ~50 units of restriction enzyme EcoRI and the resulting ~5.65 kb EcoRI fragment is isolated in substantial accordance with Example 2B.

A single transformant containing plasmid pPSJ77 (as constructed in Example 4A) is grown up and plasmid is isolated in substantial accordance with Example 1 except that 15 μg/ml tetracycline is included in the growth medium rather than 100 μg/ml ampicillin. About 25 μg of plasmid pPSJ77 (~25 μl) is mixed with 10 μl of 10 X REact™3 buffer (BRL)) 60 μl of $H_2O$, and 5 μl of restriction enzyme EcoRI (~50 units). The reaction is incubated at 37°C for 90 minutes, then sequentially extracted with phenol, phenol:chloroform, and chloroform.

The ~5.65 kb EcoRI fragment from plasmid pPS51 comprising the Aspergillus nidulans amdS gene is mixed with EcoRI digested pPSJ77 and ligated and transformed into JM109 in substantial accordance with Example 4A. The transformants are selected on plates containing 15 μg/ml tetracycline. The resulting plasmid is entitled pPSJ78. A restriction site and function map of pPSJ78 is presented in Figure 5 of the accompanying drawings. The opposite orientation of the ~5.65 kb EcoRI fragment comprising the amdS gene would work equally as well and is within the scope of the invention. The orientation of the fragment can be determined by restriction analysis with SalI (see Figure 5). The plasmid comprising the amdS gene-containing fragment in the opposite orientation is entitled pPSJ78A. A protocol for transforming Penicillium with vectors of the invention is set forth in Example 5.

Example 5

## Genetic Transformation of Penicillium

### A. Penicillium chrysogenum Strains

A Penicillium strain for transformation is obtained from the American Type Culture Collection, Rockville, MD 20852, under the accession number ATCC 9480. Other Penicillium chrysogenum strains or any commercial strains derived from ATCC 9480 by mutation, selection, or genetic breeding for the purpose of improved production of penicillin G or penicillin V are also suitable for use in preparing transformants with the vectors and plasmids of the present invention.

### B. Preparation of Uniform Inoculum for Cell Culture

To transform Penicillium chrysogenum cells efficiently, it is necessary to remove the cell walls to form stable protoplasts. In the preparation of such protoplasts it is advantageous to begin with a uniform inoculum. Otherwise, preparation of cells in culture is not reproducible and time is lost by attempts to prepare P. chrysogenum protoplasts from unsuitable or inadequate amounts of cells.

An ampoule of vegetative cells (~$10^9$ colony forming units in 1.0 ml of preservation menstruum: 5% lactose, 10% glycerol, and 0.1% Tween 80), either lyophilized or taken from liquid nitrogen storage and thawed at room temperature, are diluted in 1.0 ml of sterile saline. About 0.1 ml of this suspension is used to inoculate each of approximately 20 slants of sporulation medium: Lactose, 15.0 g/L; corn steep liquor, 2.5 g/L; peptone, 5.0 g/L; NaCl, 4.0 g/L; $MgSO_4 \cdot 7H_20$, 0.5 g/L; $KH_2PO_4$, 0.6 g/L; $FeCl_3 \cdot 6H_20$, 0.005 g/L; $CuS0_4 \cdot 5H_20$, 0.002 g/L; adjust to pH = 7.0; agar, 30.0 g/L; and autoclave 20 minutes at 120 psi.

Each slant [15 cm x 2.5 cm] contains 25 ml of solidified medium. Inoculum, spread evenly over the surface of the agar slant, is allowed to grow at 25 °C until a confluent lawn of mycelium is present and sporulated (1 week for most strains). The growth from 1 slant is suspended in 10 ml of sterile aqueous culture medium, and the suspension is transferred to 106 ml of aqueous culture medium. The flask containing the suspended cells is placed on a gyratory shaker and incubated at 25 °C for 18 hours at 285 rpm with a 1 inch throw.

Aqueous culture medium was prepared as follows: 100 ml of solution A (Sucrose, 36 g/L; L-asparagine, 7.5 g/L; $KH_2PO_4$, 15 g/L; $K_2HPO_4$, 21 g/L; $NaSO_4$, 0.75 g/L, $MgSO_4 \cdot 7H_2O$, 0.18 g/L; $CaCl_2$, 0.06 g/L; salts solution, 1 ml/L; and natural pH) are dispensed into a 500 ml shake flask; the flask is covered with a commercial closure and autoclaved at 121 °C for 20 minutes. Two ml of solution B (Glucose, 108 g/L) and 4 ml of solution C (Sucrose, 25 g/L; corn steep liquor (4% w/v nitrogen), 12.5 ml; ammonium acetate, 5.5 g/L; $CaCO_3$, 5 g/L; pH adjusted to 6.5 with KOH; and autoclaved at 121 °C for 20 minutes) are then added to solution A to prepare the aqueous culture medium.

### C. Preparation of Penicillium protoplasts

Cells from a 24 hour culture are harvested by suction filtration (Whatman #1 paper in a Buchner funnel) and suspended in buffer (0.01 M Tris(hydroxymethyl)aminomethane hydrochloride; 0.01 M $MgSO_4$; 0.01 M dithiothreitol; 1.00 M KCl; and pH = 7.0 with HCl). Sufficient buffer is added to obtain a final cell concentration of 1 g of cell mass per 50 ml of buffer. The cell suspension is placed on a gyratory water bath shaker in a 250 ml shake flask and incubated at 29-30 °C for 10 minutes at 140 rpm with a 1 inch throw. Dithiothreitol-treated cells are collected by centrifugation and then resuspended in 50 ml of enzyme solution (10 mg/ml Novozym, Novo industri A/B Bagsvaerd, Denmark; 0.01 M Tris(hydroxymethyl)-aminomethane hydrochloride; 0.01 M $MgSO_4$; 0.01 M dithiothreitol; 1.00 M KCl; and pH = 5.8 with HCl) in a 250 ml shake flask. This cell suspension is placed on a gyratory water-bath shaker and incubated at 29-30 °C for 15 minutes at 140 rpm with a 1 inch throw. Enzyme-treated cells are centrifuged at 1240Xg for 6 min, and the resulting pellet is resuspended in buffer (0.01 M Tris(hydroxymethyl)aminomethane hydrochloride; 0.01 M $MgSO_4$; 1.00 M KCl; and pH = 7.0 with HCl). The suspension is first centrifuged at 950Xg for 6

minutes. The resulting pellet is resuspended in the same buffer, and the suspension is centrifuged at 700Xg for 6 minutes. The resulting pellet is resuspended in 5 ml of the same buffer. This suspension contains primarily large protoplasts and osmotically fragile cells that retain some cell wall structure. Compared to the small protoplasts removed by the above procedure, the percentage of protoplasts able to regenerate cell walls and percentage of viable osmotically stable cells is higher for the large protoplasts and osmotically fragile cells in the final suspension. The suspension of cells is diluted with buffer to a concentration of ~2 x $10^8$ cells/ml.

D. Transformation Procedure

For each transforming plasmid, an ~0.1 ml suspension of osmotically fragile Penicillium chrysogenum cells (approximately 2 x $10^7$ cells) is supplemented with 10 $\mu$l of 50 mM $CaCl_2$, 25 $\mu$g of plasmid DNA in 5-15 $\mu$l of TE buffer, and 0.9 ml of a solution of freshly dissolved polyethylene glycol 4000 (Baker, 40% weight/volume in osmotically stabilized buffer). The mixture is vortexed, allowed to stand for 10 minutes at room temperature, centrifuged at 700Xg for 2 minutes, and vortexed again. Two aliquots of 0.5 ml each are then spread on the surface of osmotically stabilized acetamide medium (1.7 g/L Yeast Nitrogen Base without amino acids and ammonium sulfate; 125 g/L sucrose, 0.738 g/L acetamide; 1.27 g/L $CaCl_2$; and 22 g/L Noble agar). To measure the total number of viable cells present in transformation mixtures, aliquots from the transformation mixture are plated on medium in which the acetamide is replaced with an equimolar amount of ammonium sulfate. Seven to ten days after transformation, transformant colonies of sufficient size to subculture are present on the acetamide medium. Abortive transformants are easily distinguished from stable transformants, because abortive transformants fail to grow upon subculture to fresh acetamide medium. Cells transformed with a plasmid containing the acetamidase gene form visible colonies in four to five days after transformation.

## E. Analysis of Penicillium Transformants

Penicillium transformants that are transformed with vectors of the invention containing the amdS gene express an acetamidase activity (the amdS gene product) not detected in extracts of the untransformed recipient P. chrysogenum strain (e.g., ATCC 9480). This activity results in the ability of the transformed strains to grow using the ammonia released by acetamide hydrolysis when no other nitrogen sources are available. The Penicillium transformants of the invention also express racemase activity.

Stable transformants carry the transforming DNA in their high molecular weight DNA. Probes, e.g., the racemase coding sequence or fragments of Aspergillus DNA that contain the acetamidase gene, hybridize to the high molecular weight DNA from these transformants even after multiple passage on non-selective medium (ammonia as nitrogen source). The transforming phenotype (production of racemase and, if an amdS vector was used, ability to grow on acetamide as sole nitrogen source) is also maintained by the transformants after passage on non-selective medium.

## Claims

### Claims for the following Contracting States : AT, BE, CH, DE, FR, GB, GR, IT, LI, NL, SE

**1.** A constructed DNA compound that encodes a protein having the isopenicillin N epimerase (racemase) activity of Streptomyces clavuligerus, of amino acid sequence:

$NH_2$-Met Ala Val Ala Asp Trp Glu Glu Ala Arg
Gly Arg Met Leu Leu Asp Pro Thr Val Val
Asn Leu Asn Thr Gly Ser Gly Gly Pro Leu
Pro Arg Ser Phe Glu Arg Val Thr Gly Phe
Arg Ala His Leu Ala Ala Glu Pro Met Asp
Phe Leu Leu Arg Glu Val Pro Ala Leu Leu
Trp Gln Ala Arg Glu Ser Leu Ala Arg Leu
Ile Gly Gly Asp Pro Leu Arg Leu Ala Leu
Ala Thr Asn Val Thr Ala Ala Val Asn Leu
Val Ala Ser Ser Leu Arg Leu Glu Ala Pro
Gly Glu Ile Leu Leu Ser Asp Asp Glu Tyr
Thr Pro Met Arg Trp Cys Trp Glu Arg Val
Ala Arg Arg His Gly Leu Glu Leu Arg Thr
Phe Arg Leu Pro Glu Leu Pro Ser Asp Pro
Ala Glu Ile Thr Ala Ala Ala Val Ala Ala
Met Gly Pro Arg Thr Arg Leu Phe Phe Phe
Ser His Val Val Ser Thr Thr Gly Leu Ile
Leu Pro Ala Ala Glu Leu Cys Glu Glu Ala
Arg Ala Arg Gly Ile Thr Thr Val Val Asp
Gly Ala His Ala Pro Gly Phe Leu Asp Leu
Asp Leu Ser Arg Ile Pro Cys Asp Phe Tyr
Ala Gly Ser Gly His Lys Trp Leu Leu Ala
Pro Thr Gly Val Gly Phe Leu His Leu Ala
Pro Gly Arg Leu Glu Glu Leu Glu Pro Thr

Gln Val Ser Trp Ala Tyr Glu Pro Pro Glu
Ala Ala Ala Arg Arg Pro Arg Ala Thr Ala
Val Gly Ser Thr Pro Gly Leu Arg Arg Leu
Glu Cys Glu Gly Thr Arg Asp Ile Cys Pro
Trp Leu Ala Thr Pro Glu Ser Ile Asp Phe
Gln Ala Glu Leu Gly Pro Gly Ala Ile Arg
Ala Arg Arg Arg Glu Leu Thr Asp His Ala
Arg Arg Leu Leu Ala Asp Arg Pro Gly Arg
Thr Leu Leu Thr Pro Asp Ser Pro Glu Leu
Ser Gly Gly Met Val Ala Tyr Arg Leu Pro
Pro Gly Thr Asp Ala Ala Glu Leu Arg Arg
Gly Leu Trp Glu Arg Phe Arg Ile Glu Ala
Ala Val Ala Glu Gln Pro Pro Gly Pro Val
Leu Arg Ile Ser Ala Asn Phe Tyr Thr Thr
Glu Glu Glu Ile Asp Arg Leu Ala Asp Ala
Leu Asp Ala Leu Thr Gly Glu End Ser Pro
Arg Leu Ala Asp Arg Leu Pro Arg Ala Val
Asp His Pro Val His Gly Leu Arg Glu Asp
Gln-COOH

wherein Ala is an alanine residue, Arg is an arginine residue, Asn is an asparagine residue, Asp is an aspartic acid residue, Cys is a cysteine residue, Gln is a glutamine residue, Glu is a glutamic acid residue, Gly is a glycine residue, His is a histidine residue, Ile is an isoleucine residue, Leu is a leucine residue, Lys is a lysine residue, Met is a methionine residue, Phe is a phenylalanine residue, Pro is a proline residue, Ser is a serine residue, Thr is a threonine residue, Trp is a tryptophan residue, Tyr is a tyrosine residue, and Val is a valine residue.

**2.** The constructed DNA compound of Claim 1 that comprises the DNA sequence:

```
   1  ATGGCGGTAG CCGACTGGGA AGAAGCCCGC GGCCGTATGC TGCTCGACCC
  51  CACCCTCCTC AACCTCAACA CCGGCTCCGG GGGACCGCTG CCGCGCAGCT
 101  TCGAGCGGGT CACCGGCTTC CGCGCCCATC TCGCGGCCGA GCCGATGGAC
 151  TTCCTGCTCC GCGAGGTCCC CGCACTGCTG TGGCAGGCGC GGGAGAGCCT
 201  CGCCCGCCTC ATCGGCGGGG ACCCGCTGCG CCTCGCCCTG GCCACCAACG
 251  TCACCGCCGC CGTCAACCTC GTCGCGTCGT CACTGCGCCT CGAAGCGCCC
 301  GGCGAGATCC TGCTCAGCGA CGACGAGTAC ACGCCCATGC GCTGGTGCTG
 351  GGAGCGGGTC GCCCGGCGGC ACGGCCTGGA GCTGAGGACG TTCCGGCTGC
 401  CCGAGCTGCC CTCGGACCCG GCCGAGATCA CCGCGGCGGC GGTCGCCGCG
 451  ATGGGACCGC GCACCCGGCT GTTCTTCTTC AGCCATGTCG TCTCCACGAC
 501  CGGGCTGATC CTGCCCGCCG CCGAACTGTG CGAGGAGGCC CGCGCACGGG
 551  GCATCACCAC CGTGGTCGAC GGCGCCCACG CACCCGGCTT CCTCGACCTC
 601  GACCTCTCCC GGATCCCCTG CGACTTCTAC GCGGGCAGCG GCCACAAATG
 651  GCTGCTCGCC CCCACCGGGG TCGGCTTTCT CCACCTCGCC CCCGGCCGCC
 701  TGGAAGAACT GGAGCCCACC CAGGTGAGCT GGGCGTACGA GCCCCCGGAG
 751  GCAGCGGCCC GCCGGCCGCG CGCGACCGCT GTCGGCAGCA CACCCGGGCT
 801  GCGCAGACTC GAATGCGAGG GGACCCGGGA CATCTGCCCC TGGCTCGCCA
 851  CACCGGAGTC GATCGACTTC CAGGCCGAGC TGGGCCCCGG GGCGATCCGC
 901  GCCCGCCGCC GCGAGCTGAC GGACCACGCG CGCCGCCTGC TCGCCGACCG
 951  CCCGGGCCGC ACGCTCCTCA CCCCCGACTC CCCGGAGCTG TCCGGCGGCA
1001  TGGTGGCCTA CCGGCTGCCC CCGGGAACCG ACGCGGCCGA GCTGCGCCGG
1051  GGGCTCTGGG AGCGCTTCCG GATCGAGGCC GCGGTGGCCG AGCAGCCGCC
1101  CGGGCCGGTG CTCCGGATCT CGGCGAACTT CTACACCACC GAAGAGGAGA
1151  TCGACCGCCT GGCGGACGCG CTGGACGCGC TGACGGGCGA GTGATCCCCC
1201  CGGCTCGCGG ACCGCCTCCC CCGCGCTGTT GACCACCCGG TTCACGGATT
1251  ACGAGAGGAT CAGTGAGAGT TG
```

wherein A is deoxyadenyl, G is deoxyguanyl, C is deoxycytidyl, and T is thymidyl.

**3.** The ~2.0 kb ClaI-StyI restriction fragment of plasmid pOW390 (NRRL B-18431).

**4.** A recombinant DNA vector that comprises the DNA sequence of Claim 1 or 2.

**5.** The recombinant DNA vector of Claim 4 that is one of the following plasmids:

pOW392 constructed by ligating the ~0.9 kb StyI-BamHI fragment of pOW390 (NRRL B-18431) with the BamHI-HincII-digested vectors M13mp18 RF DNA to give plasmid mOW390, site-specific mutagenesis of single-stranded mOW390 DNA to give plasmid mOW391, ligation of the ~0.9 kb BamHI-NcoI fragment of mOW391, the ~5.75 kb XbaI-BamHI fragment of pCZR111 (NRRL B-18249) and a double-stranded XbaI-NcoI phosphotriester-synthesised fragment to give an intermediate plasmid which was digested with BamHI and ligated with the ~2 kb BamHI fragment of pOW390.

pPSJ77 constructed by ligating the ~7.9 kb EcoRI-NcoI restriction fragment of pOW392 with ~925 bp EcoRI-NcoI fragment of pLC2 (ATCC 53334),

pPSJ78 constructed by ligating EcoRI-SalI-digested pMLC 12 DNA (NRRL B-18097) with the ~5.0 kb EcoRI-SalI fragment of p3SR2 (NRRL B-18182) to give pPS51 and inserting the ~5.65 kb EcoRI fragment of pPS51 into EcoRI-digested pPSJ77,

pPSJ78A constructed as for pPSJ78, the ~5.65 kb EcoRi fragment being in the opposite orientation,

pOW390 (NRRL B-18431),

mOW390 constructed by ligating the ~0.9 kb StyI-BamHI fragment of pOW390 with HincII-BamHI-digested vector m13mp18, or

mOW391 produced by site specific mutagenesis of mOW390.

6. A recombinant host cell transformed with a recombinant DNA vector of Claim 4.

7. The transformed host cell of Claim 6 that is E. coli K12, Penicillium, Cephalosporium, Aspergillus, or Streptomyces.

8. The transformed host cell of Claim 7 that is E. coli K12 RR1ΔM15/pOW390 or E. coli K12 JM109/pOW392.

9. The transformed host cell of Claim 7 that is Penicillium chrysogenum/pPSJ78.

**Claims for the following Contracting State : ES**

1. A method for constructing a recombinant host cell capable of expressing isopenicillin N epimerase (racemase) activity, comprising:

transforming the host cell with a recombinant DNA expression vector that comprises:

(a) a promoter and translational-activating sequence that function in the host cell; and

(b) a DNA sequence that encodes isopenicillin N epimerase (racemase) activity operably linked to the promoter and translational activating sequence, said DNA sequence encoding a proteing have the amino acid residue sequence:

$NH_2$-Met Ala Val Ala Asp Trp Glu Glu Ala Arg
Gly Arg Met Leu Leu Asp Pro Thr Val Val
Asn Leu Asn Thr Gly Ser Gly Gly Pro Leu
Pro Arg Ser Phe Glu Arg Val Thr Gly Phe
Arg Ala His Leu Ala Ala Glu Pro Met Asp
Phe Leu Leu Arg Glu Val Pro Ala Leu Leu
Trp Gln Ala Arg Glu Ser Leu Ala Arg Leu
Ile Gly Gly Asp Pro Leu Arg Leu Ala Leu
Ala Thr Asn Val Thr Ala Ala Val Asn Leu
Val Ala Ser Ser Leu Arg Leu Glu Ala Pro

Gly Glu Ile Leu Leu Ser Asp Asp Glu Tyr
Thr Pro Met Arg Trp Cys Trp Glu Arg Val
Ala Arg Arg His Gly Leu Glu Leu Arg Thr
Phe Arg Leu Pro Glu Leu Pro Ser Asp Pro
Ala Glu Ile Thr Ala Ala Ala Val Ala Ala
Met Gly Pro Arg Thr Arg Leu Phe Phe Phe
Ser His Val Val Ser Thr Thr Gly Leu Ile
Leu Pro Ala Ala Glu Leu Cys Glu Glu Ala
Arg Ala Arg Gly Ile Thr Thr Val Val Asp
Gly Ala His Ala Pro Gly Phe Leu Asp Leu
Asp Leu Ser Arg Ile Pro Cys Asp Phe Tyr
Ala Gly Ser Gly His Lys Trp Leu Leu Ala
Pro Thr Gly Val Gly Phe Leu His Leu Ala
Pro Gly Arg Leu Glu Glu Leu Glu Pro Thr
Gln Val Ser Trp Ala Tyr Glu Pro Pro Glu
Ala Ala Ala Arg Arg Pro Arg Ala Thr Ala
Val Gly Ser Thr Pro Gly Leu Arg Arg Leu
Glu Cys Glu Gly Thr Arg Asp Ile Cys Pro
Trp Leu Ala Thr Pro Glu Ser Ile Asp Phe
Gln Ala Glu Leu Gly Pro Gly Ala Ile Arg
Ala Arg Arg Arg Glu Leu Thr Asp His Ala
Arg Arg Leu Leu Ala Asp Arg Pro Gly Arg
Thr Leu Leu Thr Pro Asp Ser Pro Glu Leu
Ser Gly Gly Met Val Ala Tyr Arg Leu Pro
Pro Gly Thr Asp Ala Ala Glu Leu Arg Arg
Gly Leu Trp Glu Arg Phe Arg Ile Glu Ala
Ala Val Ala Glu Gln Pro Pro Gly Pro Val
Leu Arg Ile Ser Ala Asn Phe Tyr Thr Thr
Glu Glu Glu Ile Asp Arg Leu Ala Asp Ala
Leu Asp Ala Leu Thr Gly Glu End Ser Pro
Arg Leu Ala Asp Arg Leu Pro Arg Ala Val
Asp His Pro Val His Gly Leu Arg Glu Asp
Gln-COOH

wherein Ala is an alanine residue, Arg is an arginine residue, Asn is an asparagine residue, Asp is an aspartic acid residue, Cys is a cysteine residue, Gln is a glutamine residue, Glu is a glutamic acid residue, Gly is a glycine residue, His is a histidine residue, Ile is an isoleucine residue, Leu is a leucine residue, Lys is a lysine residue, Met is a methionine residue, Phe is a phenylalanine residue, Pro is a proline residue, Ser is a serine residue, Thr is a threonine residue, Try is a tryptophan residue, Tyr is a tyrosine residue, and Val is a valine residue.

**2.** The method of Claim 1 in which the DNA sequence that encodes isopenicillin N epimerase activity comprises the DNA sequence:

```
   1  ATGGCGGTAG CCGACTGGGA AGAAGCCCGC GGCCGTATGC TGCTCGACCC
  51  CACCGTCGTC AACCTCAACA CCGGCTCCGG GGGACCGCTG CCGCGCAGCT
 101  TCGAGCGGGT CACCGGCTTC CGCGCCCATC TCGCGGCCGA GCCGATGGAC
 151  TTCCTGCTCC GCGAGGTCCC CGCACTGCTG TGGCAGGCGC GGGAGAGCCT
 201  CGCCCGCCTC ATCGGCGGGG ACCCGCTGCG CCTCGCCCTG GCCACCAACG
 251  TCACCGCCGC CGTCAACCTC GTCGCGTCGT CACTGCGCCT CGAAGCGCCC
 301  GGCGAGATCC TGCTCAGCGA CGACGAGTAC ACGCCCATGC GCTGGTGCTG
 351  GGAGCGGGTC GCCCGGCGGC ACGGCCTGGA GCTGAGGACG TTCCGGCTGC
 401  CCGAGCTGCC CTCGGACCCG GCCGAGATCA CCGCGGCGGC GGTCGCCGCG
 451  ATGGGACCGC GCACCCGGCT GTTCTTCTTC AGCCATGTCG TCTCCACGAC
 501  CGGGCTGATC CTGCCCGCCG CCGAACTGTG CGAGGAGGCC CGCGCACGGG
 551  GCATCACCAC CGTGGTCGAC GGCGCCCACG CACCCGGCTT CCTCGACCTC
 601  GACCTCTCCC GGATCCCCTG CGACTTCTAC GCGGGCAGCG GCCACAAATG
 651  GCTGCTCGCC CCCACCGGGG TCGGCTTTCT CCACCTCGCC CCCGGCCGCC
 701  TGGAAGAACT GGAGCCCACC CAGGTGAGCT GGGCGTACGA GCCCCCGGAG
 751  GCAGCGGCCC GCCGGCCGCG CGCGACCGCT GTCGGCAGCA CACCCGGGCT
 801  GCGCAGACTC GAATGCGAGG GGACCCGGGA CATCTGCCCC TGGCTCGCCA
 851  CACCGGAGTC GATCGACTTC CAGGCCGAGC TGGGCCCCGG GGCGATCCGC

 901  GCCCGCCGCC GCGAGCTGAC GGACCACGCG CGCCGCCTGC TCGCCGACCG
 951  CCCGGGCCGC ACGCTCCTCA CCCCCGACTC CCCGGAGCTG TCCGGCGGCA
1001  TGGTGGCCTA CCGGCTGCCC CCGGGAACCG ACGCGGCCGA GCTGCGCCGG
1051  GGGCTCTGGG AGCGCTTCCG GATCGAGGCC GCGGTGGCCG AGCAGCCGCC
1101  CGGGCCGGTG CTCCGGATCT CGGCGAACTT CTACACCACC GAAGAGGAGA
1151  TCGACCGCCT GGCGGACGCG CTGGACGCGC TGACGGGCGA GTGATCCCCC
1201  CGGCTCGCGG ACCGCCTCCC CCGCGCTGTT GACCACCCGG TTCACGGATT
1251  ACGAGAGGAT CAGTGAGAGT TG
```

wherein A is deoxyadenyl, G is deoxyguanyl, C is deoxycytidyl, and T is thymidyl.

**3.** A method for expressing isopenicillin N epimerase activity in a recombinant host cell, comprising:

culturing the host transformed in Claim 1 or 2 under conditions that allow for expression of isopenicillin N epimerase (racemase) activity.

**4.** The method of Claim 1, 2 or 3 in which the recombinant DNA expression vector is

pOW392 constructed by ligating the ~0.9 kb StyI-BamHI fragment of pOW390 (NRRL B-18431) with the BamHI-HincII-digested vectors M13mp18 RF DNA to give plasmid mOW390, site-specific mutagenesis of single-stranded mOW390 DNA to give plasmid mOW391, ligation of the ~0.9 kb BamHI-NcoI fragment of mOW391, the ~5.75 kb XbaI-BamHI fragment of pCZR111 (NRRL B-18249) and a double-stranded XbaI-NcoI phosphotriester-synthesised fragment to give an intermediate plasmid which was digested with BamHI and ligated with the ~2 kb BamHI fragment of pOW390,

pPSJ77 constructed by ligating the ~7.9 kb EcoRI-NcoI restriction fragment of pOW392 with ~925 bp EcoRI-NcoI fragment of pLC2 (ATCC 53334),

pPSJ78 constructed by ligating EcoRI-SalI-digested pMLC 12 DNA (NRRL B-18097) with the ~5.0 kb EcoRI-SalI fragment of p3SR2 (NRRL B-18182) to give pPS51 and inserting the ~5.65 kb EcoRI fragment of pPS51 into EcoRI-digested pPSJ77, or

pPSJ78A constructed as for pPSJ78, the ~5.65 kb EcoRi fragment being in the opposite orientation,

**Patentansprüche**
**Patentansprüche für folgende Vertragsstaaten : AT, BE, CH, DE, FR, GB, GR, IT, LI, NL, SE**

1. Konstruierte DNA Verbindung, die ein Protein kodiert, das die Isopenicillin-N-Epimerase (Racemase) Aktivität von Streptomyces clavuligerus und folgende Aminosäuresequenz aufweist:

$NH_2$-Met Ala Val Ala Asp Trp Glu Glu Ala Arg
Gly Arg Met Leu Leu Asp Pro Thr Val Val
Asn Leu Asn Thr Gly Ser Gly Gly Pro Leu
Pro Arg Ser Phe Glu Arg Val Thr Gly Phe
Arg Ala His Leu Ala Ala Glu Pro Met Asp
Phe Leu Leu Arg Glu Val Pro Ala Leu Leu
Trp Gln Ala Arg Glu Ser Leu Ala Arg Leu
Ile Gly Gly Asp Pro Leu Arg Leu Ala Leu
Ala Thr Asn Val Thr Ala Ala Val Asn Leu
Val Ala Ser Ser Leu Arg Leu Glu Ala Pro
Gly Glu Ile Leu Leu Ser Asp Asp Glu Tyr
Thr Pro Met Arg Trp Cys Trp Glu Arg Val
Ala Arg Arg His Gly Leu Glu Leu Arg Thr
Phe Arg Leu Pro Glu Leu Pro Ser Asp Pro
Ala Glu Ile Thr Ala Ala Ala Val Ala Ala
Met Gly Pro Arg Thr Arg Leu Phe Phe Phe
Ser His Val Val Ser Thr Thr Gly Leu Ile
Leu Pro Ala Ala Glu Leu Cys Glu Glu Ala
Arg Ala Arg Gly Ile Thr Thr Val Val Asp
Gly Ala His Ala Pro Gly Phe Leu Asp Leu
Asp Leu Ser Arg Ile Pro Cys Asp Phe Tyr
Ala Gly Ser Gly His Lys Trp Leu Leu Ala
Pro Thr Gly Val Gly Phe Leu His Leu Ala
Pro Gly Arg Leu Glu Glu Leu Glu Pro Thr
Gln Val Ser Trp Ala Tyr Glu Pro Pro Glu
Ala Ala Ala Arg Arg Pro Arg Ala Thr Ala
Val Gly Ser Thr Pro Gly Leu Arg Arg Leu
Glu Cys Glu Gly Thr Arg Asp Ile Cys Pro
Trp Leu Ala Thr Pro Glu Ser Ile Asp Phe
Gln Ala Glu Leu Gly Pro Gly Ala Ile Arg

Ala Arg Arg Arg Glu Leu Thr Asp His Ala
Arg Arg Leu Leu Ala Asp Arg Pro Gly Arg
Thr Leu Leu Thr Pro Asp Ser Pro Glu Leu
Ser Gly Gly Met Val Ala Tyr Arg Leu Pro
Pro Gly Thr Asp Ala Ala Glu Leu Arg Arg
Gly Leu Trp Glu Arg Phe Arg Ile Glu Ala
Ala Val Ala Glu Gln Pro Pro Gly Pro Val
Leu Arg Ile Ser Ala Asn Phe Tyr Thr Thr
Glu Glu Glu Ile Asp Arg Leu Ala Asp Ala
Leu Asp Ala Leu Thr Gly Glu End Ser Pro
Arg Leu Ala Asp Arg Leu Pro Arg Ala Val
Asp His Pro Val His Gly Leu Arg Glu Asp
Gln-COOH

worin Ala für einen Alaninrest steht, Arg für einen Argininrest steht, Asn für einen Asparaginrest steht, Asp für einen Asparaginsäurerest steht, Cys für einen Cysteinrest steht, Gln für einen Glutaminrest steht, Glu für einen Glutaminsäurerest steht, Gly für einen Glycinrest steht, His für einen Histidinrest steht, Ile für einen Isoleucinrest steht, Leu für einen Leucinrest steht, Lys für einen Lysinrest steht, Met für einen Methioninrest steht, Phe für einen Phenylalaninrest steht, Pro für einen Prolinrest steht, Ser für einen Serinrest steht, Thr für einen Threoninrest steht, Trp für einen Tryptophanrest steht, Tyr für einen Tyrosinrest steht und Val für einen Valinrest steht.

**2.** Konstruierte DNA Verbindung nach Anspruch 1, die folgende DNA Sequenz enthält

1 ATGGCGGTAG CCGACTGGGA AGAAGCCCGC GGCCGTATGC TGCTCGACCC
51 CACCCTCCTC AACCTCAACA CCGGCTCCGG GGGACCGCTG CCGCGCAGCT
101 TCGAGCGGGT CACCGGCTTC CGCGCCCATC TCGCGGCCGA GCCGATGGAC
151 TTCCTGCTCC GCGAGGTCCC CGCACTGCTG TGGCAGGCGC GGGAGAGCCT
201 CGCCCGCCTC ATCGGCGGGG ACCCGCTGCG CCTCGCCCTG GCCACCAACG
251 TCACCGCCGC CGTCAACCTC GTCGCGTCGT CACTGCGCCT CGAAGCGCCC
301 GGCGAGATCC TGCTCAGCGA CGACGAGTAC ACGCCCATGC GCTGGTGCTG
351 GGAGCGGGTC GCCCGGCGGC ACGGCCTGGA GCTGAGGACG TTCCGGCTGC
401 CCGAGCTGCC CTCGGACCCG GCCGAGATCA CCGCGGCGGC GGTCGCCGCG

```
451  ATGGGACCGC GCACCCGGCT GTTCTTCTTC AGCCATGTCG TCTCCACGAC
501  CGGGCTGATC CTGCCCGCCG CCGAACTGTG CGAGGAGGCC CGCGCACGGG
551  GCATCACCAC CGTGGTCGAC GGCGCCCACG CACCCGGCTT CCTCGACCTC
601  GACCTCTCCC GGATCCCCTG CGACTTCTAC GCGGGCAGCG GCCACAAATG
651  GCTGCTCGCC CCCACCGGGG TCGGCTTTCT CCACCTCGCC CCCGGCCGCC
701  TGGAAGAACT GGAGCCCACC CAGGTGAGCT GGGCGTACGA GCCCCCGGAG
751  GCAGCGGCCC GCCGGCCGCG CGCGACCGCT GTCGGCAGCA CACCCGGGCT
801  GCGCAGACTC GAATGCGAGG GGACCCGGGA CATCTGCCCC TGGCTCGCCA
851  CACCGGAGTC GATCGACTTC CAGGCCGAGC TGGGCCCCGG GGCGATCCGC
901  GCCCGCCGCC GCGAGCTGAC GGACCACGCG CGCCGCCTGC TCGCCGACCG
951  CCCGGGCCGC ACGCTCCTCA CCCCCGACTC CCCGGAGCTG TCCGGCGGCA
1001 TGGTGGCCTA CCGGCTGCCC CCGGGAACCG ACGCGGCCGA GCTGCGCCGG
1051 GGGCTCTGGG AGCGCTTCCG GATCGAGGCC GCGGTGGCCG AGCAGCCGCC
1101 CGGGCCGGTG CTCCGGATCT CGGCGAACTT CTACACCACC GAAGAGGAGA
1151 TCGACCGCCT GGCGGACGCG CTGGACGCGC TGACGGGCGA GTGATCCCCC
1201 CGGCTCGCGG ACCGCCTCCC CCGCGCTGTT GACCACCCGG TTCACGGATT
1251 ACGAGAGGAT CAGTGAGAGT TG
```

worin A für Desoxyadenyl steht, G für Desoxyguanyl steht, C für Desoxycytidyl steht und T für Thymidyl steht.

3. ~2,0 kb ClaI-StyI Restriktionsfragment von Plasmid pOW390 (NRRL B-18431).

4. Rekombinanter DNA Vektor, der die DNA Sequenz nach Anspruch 1 oder 2 enthält.

5. Rekombinanter DNA Vektor nach Anspruch 4, der eines der folgenden Plasmide ist:

pOW392, konstruiert durch Ligation des ~0,9 kb StyI-BamHI Fragments von pOW390 (NRRL B-18431) mit der BamHI-HincII-verdauten M13mp18 RF Vektor-DNA unter Bildung von Plasmid mOW390, ortspezifische Mutagenese der einzelsträngigen mOW390 DNA unter Bildung von Plasmid mOW391, Ligation des ~0,9 kb BamHI-NcoI Fragments von mOW391, des ~5,75 kb XbaI-BamHI Fragments von pCZR111 (NRRL B-18249) und eines doppelsträngigen Phosphotriester-synthetisierten XbaI-NcoI-Fragments unter Bildung eines als Zwischenstufe benötigten Plasmids, das mit BamHI verdaut und mit dem ~2 kb BamHI Fragment von pOW390 ligiert wird,

pPSJ77, konstruiert durch Ligation des ~7,9 kb EcoRI-NcoI Reatriktionsfragments von pOW392 mit dem ~925 bp EcoRI-NcoI Fragment von pLC2 (ATCC 53334),

pPSJ78, konstruiert durch Ligation der EcoRI-SalI-verdauten pMLC 12 DNA (NRRL B-18097) mit dem ~5,0 kb EcoRI-SalI Fragment von p3SR2 (NRRL B-18182) unter Bildung von pPS51 und Insertion des ~5,65 kb EcoRI Fragments von pPS51 in das EcoRI-verdaute pPSJ77,

pPSJ78A, konserviert wie pPSJ78, wobei das ~5,65 kb EcoRI Fragment in der entgegengesetzten Richtung liegt,

pOW390 (NRRL B-18431),

mOW390, konstruiert durch Ligation des ~0,9 kb StyI-BamHI Fragments von pOW390 mit dem HincII-BamHI-verdauten Vektor M13mp18, oder

mOW391, hergestellt durch ortsspezifische Mutagenese von mOW390.

6. Rekombinante Wirtszelle, die mit einem rekombinanten DNA Vektor nach Anspruch 4 transformiert ist.

7. Transformierte Wirtszelle nach Anspruch 6, die E. coli K12, Penicillium, Cephalosporium, Aspergillus oder Streptomyces ist.

8. Transformierte Wirtszelle nach Anspruch 7, die E. coli K12 RR1ΔM15/pOW390 oder E. coli K12 JM109/pOW392 ist.

9. Transformierte Wirtszelle nach Anspruch 7, die Penicillium chrysogenum/pPSJ78 ist.

**Patentansprüche für folgenden Vertragsstaat : ES**

1. Verfahren zur Konstruktion einer rekombinanten Wirtszelle, die zur Expression einer Isopenicillin-N-Epimerase (Racemaseaktivität) fähig ist, das umfaßt:
Transformieren der Wirtszelle mit einem rekombinanten DNA Expressionsvektor, der enthält:
(a) einen Promotor und eine Translationsaktivierungssequenz, die in der Wirtszelle funktionieren, und
(b) eine DNA Sequenz, die Isopenicillin-N-Epimerase (Racemase) Aktivität kodiert, funktionsfähig mit dem Promotor und der Translationsaktivierungssequenz verbunden, wobei diese DNA Sequenz ein Protein kodiert, das die folgende Aminosäuresequenz aufweist:

$NH_2$-Met Ala Val Ala Asp Trp Glu Glu Ala Arg
Gly Arg Met Leu Leu Asp Pro Thr Val Val
Asn Leu Asn Thr Gly Ser Gly Gly Pro Leu
Pro Arg Ser Phe Glu Arg Val Thr Gly Phe
Arg Ala His Leu Ala Ala Glu Pro Met Asp
Phe Leu Leu Arg Glu Val Pro Ala Leu Leu
Trp Gln Ala Arg Glu Ser Leu Ala Arg Leu
Ile Gly Gly Asp Pro Leu Arg Leu Ala Leu
Ala Thr Asn Val Thr Ala Ala Val Asn Leu
Val Ala Ser Ser Leu Arg Leu Glu Ala Pro
Gly Glu Ile Leu Leu Ser Asp Asp Glu Tyr
Thr Pro Met Arg Trp Cys Trp Glu Arg Val
Ala Arg Arg His Gly Leu Glu Leu Arg Thr
Phe Arg Leu Pro Glu Leu Pro Ser Asp Pro
Ala Glu Ile Thr Ala Ala Ala Val Ala Ala
Met Gly Pro Arg Thr Arg Leu Phe Phe Phe
Ser His Val Val Ser Thr Thr Gly Leu Ile
Leu Pro Ala Ala Glu Leu Cys Glu Glu Ala
Arg Ala Arg Gly Ile Thr Thr Val Val Asp
Gly Ala His Ala Pro Gly Phe Leu Asp Leu

Asp Leu Ser Arg Ile Pro Cys Asp Phe Tyr
Ala Gly Ser Gly His Lys Trp Leu Leu Ala
Pro Thr Gly Val Gly Phe Leu His Leu Ala
Pro Gly Arg Leu Glu Glu Leu Glu Pro Thr
Gln Val Ser Trp Ala Tyr Glu Pro Pro Glu
Ala Ala Ala Arg Arg Pro Arg Ala Thr Ala
Val Gly Ser Thr Pro Gly Leu Arg Arg Leu
Glu Cys Glu Gly Thr Arg Asp Ile Cys Pro
Trp Leu Ala Thr Pro Glu Ser Ile Asp Phe
Gln Ala Glu Leu Gly Pro Gly Ala Ile Arg
Ala Arg Arg Arg Glu Leu Thr Asp His Ala
Arg Arg Leu Leu Ala Asp Arg Pro Gly Arg
Thr Leu Leu Thr Pro Asp Ser Pro Glu Leu
Ser Gly Gly Met Val Ala Tyr Arg Leu Pro
Pro Gly Thr Asp Ala Ala Glu Leu Arg Arg
Gly Leu Trp Glu Arg Phe Arg Ile Glu Ala
Ala Val Ala Glu Gln Pro Pro Gly Pro Val
Leu Arg Ile Ser Ala Asn Phe Tyr Thr Thr
Glu Glu Glu Ile Asp Arg Leu Ala Asp Ala
Leu Asp Ala Leu Thr Gly Glu End Ser Pro
Arg Leu Ala Asp Arg Leu Pro Arg Ala Val
Asp His Pro Val His Gly Leu Arg Glu Asp
Gln-COOH

worin Ala für einen Alaninrest steht, Arg für einen Argininrest steht, Asn für einen Asparaginrest steht, Asp für einen Asparaginsäurerest steht, Cys für einen Cysteinrest steht, Gln für einen Glutaminrest steht, Glu für einen Glutaminsäurerest steht, Gly für einen Glycinrest steht, His für einen Histidinrest steht, Ile für einen Isoleucinrest steht, Leu für einen Leucinrest steht, Lys für einen Lysinrest steht, Met für einen Methioninrest steht, Phe für einen Phenylalaninrest steht, Pro für einen Prolinrest steht, Ser für einen Serinrest steht, Thr für einen Threoninrest steht, Trp für einen Tryptophanrest steht, Tyr für einen Tyrosinrest steht und Val für einen Valinrest steht.

**2.** Verfahren nach Anspruch 1, worin die DNA Sequenz, die die Isopenicillin-N-Epimeraseaktivität kodiert, die folgende DNA Sequenz enthält

```
   1 ATGGCGGTAG CCGACTGGGA AGAAGCCCGC GGCCGTATGC TGCTCGACCC
  51 CACCGTCGTC AACCTCAACA CCGGCTCCGG GGGACCGCTG CCGCGCAGCT
 101 TCGAGCGGGT CACCGGCTTC CGCGCCCATC TCGCGGCCGA GCCGATGGAC
 151 TTCCTGCTCC GCGAGGTCCC CGCACTGCTG TGGCAGGCGC GGGAGAGCCT
 201 CGCCCGCCTC ATCGGCGGGG ACCCGCTGCG CCTCGCCCTG GCCACCAACG
 251 TCACCGCCGC CGTCAACCTC GTCGCGTCGT CACTGCGCCT CGAAGCGCCC
 301 GGCGAGATCC TGCTCAGCGA CGACGAGTAC ACGCCCATGC GCTGGTGCTG
 351 GGAGCGGGTC GCCCGGCGGC ACGGCCTGGA GCTGAGGACG TTCCGGCTGC
 401 CCGAGCTGCC CTCGGACCCG GCCGAGATCA CCGCGGCGGC GGTCGCCGCG
 451 ATGGGACCGC GCACCCGGCT GTTCTTCTTC AGCCATGTCG TCTCCACGAC
 501 CGGGCTGATC CTGCCCGCCG CCGAACTGTG CGAGGAGGCC CGCGCACGGG
 551 GCATCACCAC CGTGGTCGAC GGCGCCCACG CACCCGGCTT CCTCGACCTC
 601 GACCTCTCCC GGATCCCCTG CGACTTCTAC GCGGGCAGCG GCCACAAATG
 651 GCTGCTCGCC CCCACCGGGG TCGGCTTTCT CCACCTCGCC CCCGGCCGCC
 701 TGGAAGAACT GGAGCCCACC CAGGTGAGCT GGGCGTACGA GCCCCCGGAG
 751 GCAGCGGCCC GCCGGCCGCG CGCGACCGCT GTCGGCAGCA CACCCGGGCT
 801 GCGCAGACTC GAATGCGAGG GGACCCGGGA CATCTGCCCC TGGCTCGCCA
 851 CACCGGAGTC GATCGACTTC CAGGCCGAGC TGGGCCCCGG GGCGATCCGC
 901 GCCCGCCGCC GCGAGCTGAC GGACCACGCG CGCCGCCTGC TCGCCGACCG
 951 CCCGGGCCGC ACGCTCCTCA CCCCCGACTC CCCGGAGCTG TCCGGCGGCA
1001 TGGTGGCCTA CCGGCTGCCC CCGGGAACCG ACGCGGCCGA GCTGCGCCGG
1051 GGGCTCTGGG AGCGCTTCCG GATCGAGGCC GCGGTGGCCG AGCAGCCGCC
1101 CGGGCCGGTG CTCCGGATCT CGGCGAACTT CTACACCACC GAAGAGGAGA
1151 TCGACCGCCT GGCGGACGCG CTGGACGCGC TGACGGGCGA GTGATCCCCC
1201 CGGCTCGCGG ACCGCCTCCC CCGCGCTGTT GACCACCCGG TTCACGGATT
1251 ACGAGAGGAT CAGTGAGAGT TG
```

worin A für Desoxyadenyl steht, G für Desoxyguanyl steht, C für Desoxycytidyl steht und T für Thymidyl steht.

3. Verfahren zur Expression der Isopenicillin-N-Epimeraseaktivität in einer rekombinanten Wirtszelle, das umfaßt: Kultivieren einer nach Anspruch 1 oder 2 transformierten Wirtszelle unter Bedingungen, die die Expression der Isopenicillin-N-Epimerase (Racemase) Aktivität erlauben.

4. Verfahren nach Anspruch 1, 2 oder 3, worin der rekombinante DNA Expressionsvektor ist

pOW392, konstruiert durch Ligation des ~0,9 kb StyI-BamHI Fragments von pOW390 (NRRL B-18431) mit der BamHI-HincII-verdauten M13mp18 RF Vektor-DNA unter Bildung von Plasmid mOW390, ortspezifische Mutagenese der einzelsträngigen mOW390 DNA unter Bildung von Plasmid mOW391, Ligation des ~0,9 kb BamHI-NcoI Fragments von mOW391, des ~5,75 kb XbaI-BamHI Fragments von pCZR111 (NRRL B-18249) und eines doppelsträngigen Phosphotriester-synthetisierten XbaI-NcoI Fragments unter Bildung eines als Zwischenstufe benötigten Plasmids, das mit BamHI verdaut und mit dem ~2 kb BamHI Fragment von pOW390 ligiert wird,

pPSJ77, konstruiert durch Ligation des ~7,9 kb EcoRI-NcoI Restriktionsfragments von pOW392 mit dem ~925 bp EcoRI-NcoI Fragment von pLC2 (ATCC 53334),

pPSJ78, konstruiert durch Ligation der EcoRI-SalI-verdauten pMLC 12 DNA (NRRL B-18097) mit dem ~5,0 kb EcoRI-SalI Fragment von p3SR2 (NRRL B-18182) unter Bildung von pPS51 und Insertion des ~5,65 kb EcoRI Fragments von pPS51 in das EcoRI-verdaute pPSJ77, oder

pPSJ78A, konstruiert wie pPSJ78, wobei das ~5,65 kb EcoRI Fragment in der entgegengesetzten Richtung liegt.

**Revendications**

**Revendications pour les Etats contractants suivants : AT, BE, CH, DE, FR, GB, GR, IT, LI, NL, SE**

1. Composé d'ADN construit qui code pour une protéine ayant l'activité de l'isopénicilline N épimérase (racémase) de Streptomyces clavuligerus, présentant la séquence d'acides aminés :

$NH_2$-Met Ala Val Ala Asp Trp Glu Glu Ala Arg
Gly Arg Met Leu Leu Asp Pro Thr Val Val
Asn Leu Asn Thr Gly Ser Gly Gly Pro Leu
Pro Arg Ser Phe Glu Arg Val Thr Gly Phe
Arg Ala His Leu Ala Ala Glu Pro Met Asp
Phe Leu Leu Arg Glu Val Pro Ala Leu Leu
Trp Gln Ala Arg Glu Ser Leu Ala Arg Leu
Ile Gly Gly Asp Pro Leu Arg Leu Ala Leu
Ala Thr Asn Val Thr Ala Ala Val Asn Leu
Val Ala Ser Ser Leu Arg Leu Glu Ala Pro
Gly Glu Ile Leu Leu Ser Asp Asp Glu Tyr
Thr Pro Met Arg Trp Cys Trp Glu Arg Val
Ala Arg Arg His Gly Leu Glu Leu Arg Thr
Phe Arg Leu Pro Glu Leu Pro Ser Asp Pro
Ala Glu Ile Thr Ala Ala Ala Val Ala Ala
Met Gly Pro Arg Thr Arg Leu Phe Phe Phe
Ser His Val Val Ser Thr Thr Gly Leu Ile
Leu Pro Ala Ala Glu Leu Cys Glu Glu Ala
Arg Ala Arg Gly Ile Thr Thr Val Val Asp
Gly Ala His Ala Pro Gly Phe Leu Asp Leu
Asp Leu Ser Arg Ile Pro Cys Asp Phe Tyr
Ala Gly Ser Gly His Lys Trp Leu Leu Ala
Pro Thr Gly Val Gly Phe Leu His Leu Ala
Pro Gly Arg Leu Glu Glu Leu Glu Pro Thr

Gln Val Ser Trp Ala Tyr Glu Pro Pro Glu
Ala Ala Ala Arg Arg Pro Arg Ala Thr Ala
Val Gly Ser Thr Pro Gly Leu Arg Arg Leu
Glu Cys Glu Gly Thr Arg Asp Ile Cys Pro
Trp Leu Ala Thr Pro Glu Ser Ile Asp Phe
Gln Ala Glu Leu Gly Pro Gly Ala Ile Arg
Ala Arg Arg Arg Glu Leu Thr Asp His Ala
Arg Arg Leu Leu Ala Asp Arg Pro Gly Arg
Thr Leu Leu Thr Pro Asp Ser Pro Glu Leu
Ser Gly Gly Met Val Ala Tyr Arg Leu Pro
Pro Gly Thr Asp Ala Ala Glu Leu Arg Arg
Gly Leu Trp Glu Arg Phe Arg Ile Glu Ala
Ala Val Ala Glu Gln Pro Pro Gly Pro Val
Leu Arg Ile Ser Ala Asn Phe Tyr Thr Thr
Glu Glu Glu Ile Asp Arg Leu Ala Asp Ala
Leu Asp Ala Leu Thr Gly Glu End Ser Pro
Arg Leu Ala Asp Arg Leu Pro Arg Ala Val
Asp His Pro Val His Gly Leu Arg Glu Asp
Gln-COOH

dans laquelle Ala représente un résidu d'alanine Arg représente un résidu d'arginine, Asn représente un résidu d'asparagine, Asp représente un résidu d'acide aspartique, Cys représente un résidu de cystéine, Gln représente un résidu de glutamine, Glu représente un résidu d'acide glutamique, Gly représente un résidu de glycine, His représente un résidu d'hystidine, Ile représente un résidu d'isoleucine, Leu représente un résidu de leucine, Lys représente un résidu de lysine, Met représente un résidu de méthionine, Phe représente un résidu de phénylalanine, Pro représente un résidu de proline, Ser représente un résidu de sérine, Thr représente un résidu de thréonine, Trp représente un résidu de tryptophane, Tyr représente un résidu de tyrosine et Val représente un résidu de valine.

**2.** Composé d'ADN construit selon la revendication 1 qui comprend la séquence d'ADN :

```
   1 ATGGCGGTAG CCGACTGGGA AGAAGCCCGC GGCCGTATGC TGCTCGACCC
  51 CACCCTCCTC AACCTCAACA CCGGCTCCGG GGGACCGCTG CCGCGCAGCT
 101 TCGAGCGGGT CACCGGCTTC CGCGCCCATC TCGCGGCCGA GCCGATGGAC
 151 TTCCTGCTCC GCGAGGTCCC CGCACTGCTG TGGCAGGCGC GGGAGAGCCT
 201 CGCCCGCCTC ATCGGCGGGG ACCCGCTGCG CCTCGCCCTG GCCACCAACG
 251 TCACCGCCGC CGTCAACCTC GTCGCGTCGT CACTGCGCCT CGAAGCGCCC
 301 GGCGAGATCC TGCTCAGCGA CGACGAGTAC ACGCCCATGC GCTGGTGCTG
 351 GGAGCGGGTC GCCCGGCGGC ACGGCCTGGA GCTGAGGACG TTCCGGCTGC
 401 CCGAGCTGCC CTCGGACCCG GCCGAGATCA CCGCGGCGGC GGTCGCCGCG
 451 ATGGGACCGC GCACCCGGCT GTTCTTCTTC AGCCATGTCG TCTCCACGAC
 501 CGGGCTGATC CTGCCCGCCG CCGAACTGTG CGAGGAGGCC CGCGCACGGG
 551 GCATCACCAC CGTGGTCGAC GGCGCCCACG CACCCGGCTT CCTCGACCTC
 601 GACCTCTCCC GGATCCCCTG CGACTTCTAC GCGGGCAGCG GCCACAAATG
 651 GCTGCTCGCC CCCACCGGGG TCGGCTTTCT CCACCTCGCC CCCGGCCGCC
 701 TGGAAGAACT GGAGCCCACC CAGGTGAGCT GGGCGTACGA GCCCCCGGAG
 751 GCAGCGGCCC GCCGGCCGCG CGCGACCGCT GTCGGCAGCA CACCCGGGCT
 801 GCGCAGACTC GAATGCGAGG GGACCCGGGA CATCTGCCCC TGGCTCGCCA
 851 CACCGGAGTC GATCGACTTC CAGGCCGAGC TGGGCCCCGG GGCGATCCGC
 901 GCCCGCCGCC GCGAGCTGAC GGACCACGCG CGCCGCCTGC TCGCCGACCG
 951 CCCGGGCCGC ACGCTCCTCA CCCCCGACTC CCCGGAGCTG TCCGGCGGCA
1001 TGGTGGCCTA CCGGCTGCCC CCGGGAACCG ACGCGGCCGA GCTGCGCCGG
1051 GGGCTCTGGG AGCGCTTCCG GATCGAGGCC GCGGTGGCCG AGCAGCCGCC
1101 CGGGCCGGTG CTCCGGATCT CGGCGAACTT CTACACCACC GAAGAGGAGA
1151 TCGACCGCCT GGCGGACGCG CTGGACGCGC TGACGGGCGA GTGATCCCCC
1201 CGGCTCGCGG ACCGCCTCCC CCGCGCTGTT GACCACCCGG TTCACGGATT
1251 ACGAGAGGAT CAGTGAGAGT TG
```

dans laquelle A représente le désoxyadényle, G représente le désoxyguanyle, C représente le désoxycytidyle et T représente le thymidyle.

**3.** Fragment de restriction ClaI-StyI d'environ 2,0 kb du plasmide pOW390 (NRRL B-18431).

**4.** Vecteur d'ADN recombinant qui comprend la séquence d'ADN selon la revendication 1 ou 2.

**5.** Vecteur d'ADN recombinant selon la revendication 4, qui est un des plasmides suivants :
pOW392 construit en liant le fragment StyI-BamHI d'environ 0,9 kb de pOW390 (NRRL B-18431) avec l'ADN RF des vecteurs M13mp18 digérés par BamHI-HincII pour donner le plasmide mOW390, la mutagenèse spécifique au site de l'ADN de mOW390 simple-brin pour donner le plasmide mOW391, la liaison du fragment BamHI-NcoI d'environ 0,9 kb de mOW391, le fragment XbaI-BamHI d'environ 5,75 kb de pCZR111 (NRRL B-18249) et un fragment XbaI-NcoI double-brins synthétisé par la méthode du

phosphotriester pour donner un plasmide intermédiaire qui a été digéré par BamHI et lié avec le fragment BamHI d'environ 2 kb de pOW390,

pPSJ77 construit en liant le fragment de restriction EcoRI-NcoI d'environ 7,9 kb de pOW392 avec le fragment EcoRI-NcoI d'environ 925 pb de pLC2 (ATCC 53334),

pPSJ78 construit en liant l'ADN de pMLC 12 digéré par EcoRI-SalI (NRRL B-18097) avec le fragment EcoRI-SalI d'environ 5,0 kb de p3SR2 (NRRL B-18182) de façon à donner le pPS51 et en insérant le fragment EcoRI d'environ 5,65 kb de pPS51 dans le pPSJ77 digéré par EcoRI,

pPSJ78A construit de la même façon que le pPSJ78, le fragment EcoRI d'environ 5,65 kb se trouvant dans l'orientation opposée,

pOW390 (NRRL B-18431),

mOW390 construit en liant le fragment StyI-BamHI d'environ 0,9 kb du pOW390 avec le vecteur m13mp18 digéré par HincII-BamHI, ou

mOW391 produit par la mutagenèse spécifique au site de mOW390.

**6.** Cellule-hôte recombinante transformée avec un vecteur d'ADN recombinant selon la revendication 4.

**7.** Cellule-hôte transformée selon la revendication 6, qui est E. coli K12, Penicillium, Cephalosporium, Aspergillus ou Streptomyces.

**8.** Cellule-hôte transformée selon la revendication 7, qui est E. coli K12 RR1ΔM15/pOW390 ou E. coli K12 JM109/pOW392.

**9.** Cellule-hôte transformée selon la revendication 7, qui est Penicillium chrysogenum/pPSJ78.

**Revendications pour l'Etat contractant suivant : ES**

**1.** Méthode pour la construction d'une cellule-hôte recombinante capable d'exprimer une activité d'isopénicilline N épimérase (racémase), comprenant :

la transformation de la cellule-hôte avec un vecteur d'expression d'ADN recombinant qui comprend :

(a) un promoteur et une séquence d'activation de la traduction qui fonctionne dans la cellule-hôte; et

(b) une séquence d'ADN qui code pour une activité de l'isopénicilline N épimérase (racémase) liée de manière fonctionnelle au promoteur et à la séquence d'activation de la traduction, ladite séquence d'ADN codant pour une protéine qui présente la sèquence de résidus d'acides aminés :

$NH_2$-Met Ala Val Ala Asp Trp Glu Glu Ala Arg
Gly Arg Met Leu Leu Asp Pro Thr Val Val
Asn Leu Asn Thr Gly Ser Gly Gly Pro Leu
Pro Arg Ser Phe Glu Arg Val Thr Gly Phe
Arg Ala His Leu Ala Ala Glu Pro Met Asp
Phe Leu Leu Arg Glu Val Pro Ala Leu Leu
Trp Gln Ala Arg Glu Ser Leu Ala Arg Leu
Ile Gly Gly Asp Pro Leu Arg Leu Ala Leu
Ala Thr Asn Val Thr Ala Ala Val Asn Leu
Val Ala Ser Ser Leu Arg Leu Glu Ala Pro

Gly Glu Ile Leu Leu Ser Asp Asp Glu Tyr
Thr Pro Met Arg Trp Cys Trp Glu Arg Val
Ala Arg Arg His Gly Leu Glu Leu Arg Thr
Phe Arg Leu Pro Glu Leu Pro Ser Asp Pro
Ala Glu Ile Thr Ala Ala Ala Val Ala Ala
Met Gly Pro Arg Thr Arg Leu Phe Phe Phe
Ser His Val Val Ser Thr Thr Gly Leu Ile
Leu Pro Ala Ala Glu Leu Cys Glu Glu Ala
Arg Ala Arg Gly Ile Thr Thr Val Val Asp
Gly Ala His Ala Pro Gly Phe Leu Asp Leu
Asp Leu Ser Arg Ile Pro Cys Asp Phe Tyr
Ala Gly Ser Gly His Lys Trp Leu Leu Ala
Pro Thr Gly Val Gly Phe Leu His Leu Ala
Pro Gly Arg Leu Glu Glu Leu Glu Pro Thr
Gln Val Ser Trp Ala Tyr Glu Pro Pro Glu
Ala Ala Ala Arg Arg Pro Arg Ala Thr Ala
Val Gly Ser Thr Pro Gly Leu Arg Arg Leu
Glu Cys Glu Gly Thr Arg Asp Ile Cys Pro
Trp Leu Ala Thr Pro Glu Ser Ile Asp Phe
Gln Ala Glu Leu Gly Pro Gly Ala Ile Arg
Ala Arg Arg Arg Glu Leu Thr Asp His Ala
Arg Arg Leu Leu Ala Asp Arg Pro Gly Arg
Thr Leu Leu Thr Pro Asp Ser Pro Glu Leu
Ser Gly Gly Met Val Ala Tyr Arg Leu Pro
Pro Gly Thr Asp Ala Ala Glu Leu Arg Arg
Gly Leu Trp Glu Arg Phe Arg Ile Glu Ala
Ala Val Ala Glu Gln Pro Pro Gly Pro Val
Leu Arg Ile Ser Ala Asn Phe Tyr Thr Thr
Glu Glu Glu Ile Asp Arg Leu Ala Asp Ala
Leu Asp Ala Leu Thr Gly Glu End Ser Pro
Arg Leu Ala Asp Arg Leu Pro Arg Ala Val
Asp His Pro Val His Gly Leu Arg Glu Asp
Gln-COOH

dans laquelle Ala représente un résidu d'alanine, Arg représente un résidu d'arginine, Asn représente un résidu d'asparagine, Asp représente un résidu d'acide aspartique, Cys représente un résidu de cystéine, Gln représente un résidu de glutamine, Glu représente un résidu d'acide glutamique, Gly représente un résidu de glycine, His représente un résidu d'hystidine, Ile représente un résidu d'isoleucine, Leu représente un résidu de leucine, Lys représente un résidu de lysine, Met

représente un résidu de méthionine, Phe représente un résidu de phénylalanine, Pro représente un résidu de proline, Ser représente un résidu de sérine, Thr représente un résidu de thréonine, Trp représente un résidu de tryptophane, Tyr représente un résidu de tyrosine et Val représente un résidu de valine.

**2.** Méthode selon la revendication 1, dans laquelle la séquence d'ADN qui code pour une activité d'isopénicilline N épimérase comprend la séquence d'ADN :

```
   1 ATGGCGGTAG CCGACTGGGA AGAAGCCCGC GGCCGTATGC TGCTCGACCC
  51 CACCGTCGTC AACCTCAACA CCGGCTCCGG GGGACCGCTG CCGCGCAGCT
 101 TCGAGCGGGT CACCGGCTTC CGCGCCCATC TCGCGGCCGA GCCGATGGAC
 151 TTCCTGCTCC GCGAGGTCCC CGCACTGCTG TGGCAGGCGC GGGAGAGCCT
 201 CGCCCGCCTC ATCGGCGGGG ACCCGCTGCG CCTCGCCCTG GCCACCAACG
 251 TCACCGCCGC CGTCAACCTC GTCGCGTCGT CACTGCGCCT CGAAGCGCCC
 301 GGCGAGATCC TGCTCAGCGA CGACGAGTAC ACGCCCATGC GCTGGTGCTG
 351 GGAGCGGGTC GCCCGGCGGC ACGGCCTGGA GCTGAGGACG TTCCGGCTGC
 401 CCGAGCTGCC CTCGGACCCG GCCGAGATCA CCGCGGCGGC GGTCGCCGCG
 451 ATGGACCGC GCACCCGGCT GTTCTTCTTC AGCCATGTCG TCTCCACGAC
 501 CGGGCTGATC CTGCCCGCCG CCGAACTGTG CGAGGAGGCC CGCGCACGGG
 551 GCATCACCAC CGTGGTCGAC GGCGCCCACG CACCCGGCTT CCTCGACCTC
 601 GACCTCTCCC GGATCCCCTG CGACTTCTAC GCGGGCAGCG GCCACAAATG
 651 GCTGCTCGCC CCCACCGGGG TCGGCTTTCT CCACCTCGCC CCCGGCCGCC
 701 TGGAAGAACT GGAGCCCACC CAGGTGAGCT GGGCGTACGA GCCCCCGGAG
 751 GCAGCGGCCC GCCGGCCGCG CGCGACCGCT GTCGGCAGCA CACCCGGGCT
 801 GCGCAGACTC GAATGCGAGG GGACCCGGGA CATCTGCCCC TGGCTCGCCA
 851 CACCGGAGTC GATCGACTTC CAGGCCGAGC TGGGCCCCGG GGCGATCCGC

 901 GCCCGCCGCC GCGAGCTGAC GGACCACGCG CGCCGCCTGC TCGCCGACCG
 951 CCCGGGCCGC ACGCTCCTCA CCCCCGACTC CCCGGAGCTG TCCGGCGGCA
1001 TGGTGGCCTA CCGGCTGCCC CCGGGAACCG ACGCGGCCGA GCTGCGCCGG
1051 GGGCTCTGGG AGCGCTTCCG GATCGAGGCC GCGGTGGCCG AGCAGCCGCC
1101 CGGGCCGGTG CTCCGGATCT CGGCGAACTT CTACACCACC GAAGAGGAGA
1151 TCGACCGCCT GGCGGACGCG CTGGACGCGC TGACGGGCGA GTGATCCCCC
1201 CGGCTCGCGG ACCGCCTCCC CCGCGCTGTT GACCACCCGG TTCACGGATT
1251 ACGAGAGGAT CAGTGAGAGT TG
```

dans laquelle A représente le désoxyadényle, G représente le désoxyguanyle, C représente le désoxycytidyle et T représente le thymidyle.

**3.** Méthode pour exprimer une activité d'isopénicilline N épimérase dans une cellule-hôte recombinante, comprenant :

la culture de l'hôte transformè à la revendication 1 ou 2 dans des conditions qui permettent

l'expression de l'activité d'isopénicilline N épimérase (racémase).

**4.** Méthode selon la revendication 1, 2 ou 3, dans laquelle le vecteur d'expression d'ADN recombinant est

pOW392 construit en liant le fragment StyI-BamHI d'environ 0,9 kb de pOW390 (NRRL B-18431) avec l'ADN RE des vecteurs M13mp18 digérés par BamHI-HincII pour donner le plasmide mOW390, la mutagenèse spécifique au site de l'ADN de mOW390 simple-brin pour donner le plasmide mOW391, la liaison du fragment BamHI-NcoI d'environ 0,9 kb de mOW391, le fragment XbaI-BamHI d'environ 5,75 kb de pCZR111 (NRRL B-18249) et un fragment XbaI-NcoI double-brins synthétisé par la méthode du phosphotriester pour donner un plasmide intermédiaire qui a été digéré par BamHI et lié avec le fragment BamHI d'environ 2 kb de pOW390,

pPSJ77 construit en liant le fragment de restriction EcoRI-NcoI d'environ 7,9 kb de pOW392 avec le fragment EcoRI-NcoI d'environ 925 pb de pLC2 (ATCC 53334),

pPSJ78 construit en liant l'ADN de pMLC12 digéré par EcoRI-SalI (NRRL B-18097) avec le fragment EcoRI-SalI d'environ 5,0 kb de p3SR2 (NRRL B-18182) de façon à donner le pPS51 et en insérant le fragment EcoRI d'environ 5,65 kb de pPS51 dans le pPSJ77 digéré par EcoRI, ou

pPSJ78A construit de la même façon que le pPSJ78, le fragment EcoRI d'environ 5,65 kb se trouvant dans l'orientation opposée.

FIG. 1
**Restriction Site and Function Map of Plasmid pOW390 (~6.2 kb)**

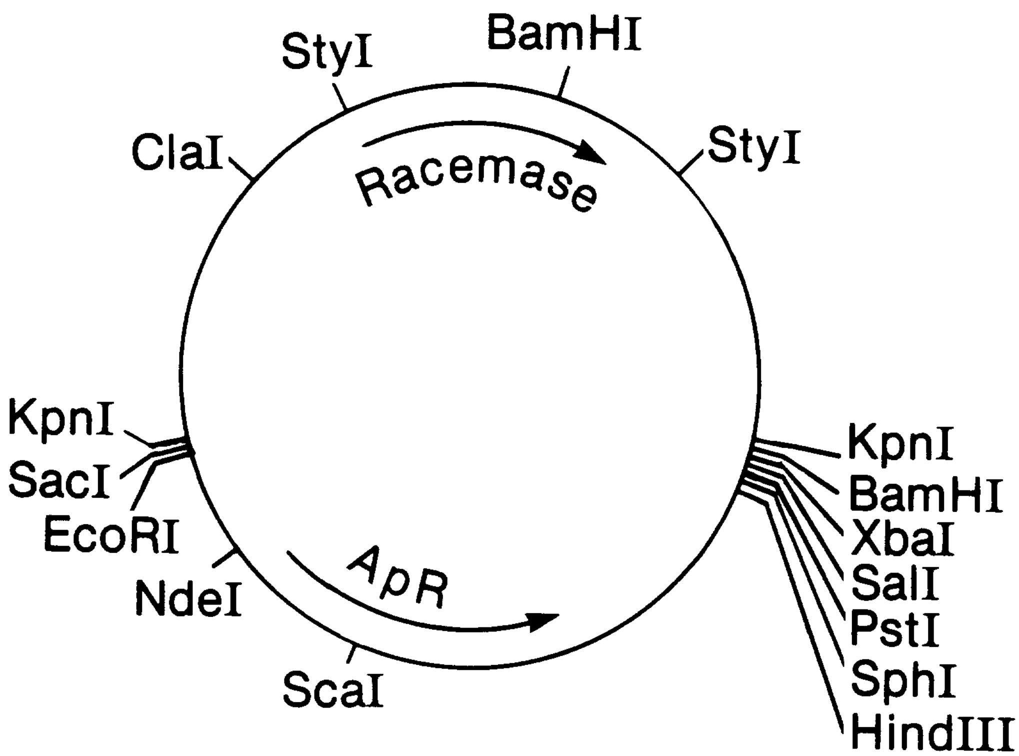

FIG.2

# Restriction Site and Function Map of Plasmid pCZR111 (6,395 bp)

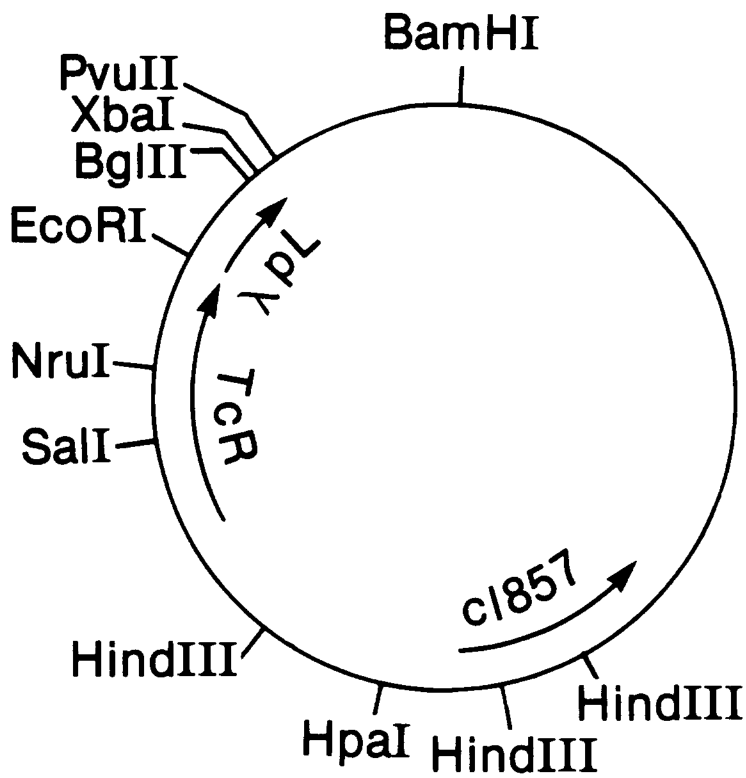

FIG.3

**Restriction Site and Function Map of Plasmid pOW392 (~8.4 kb)**

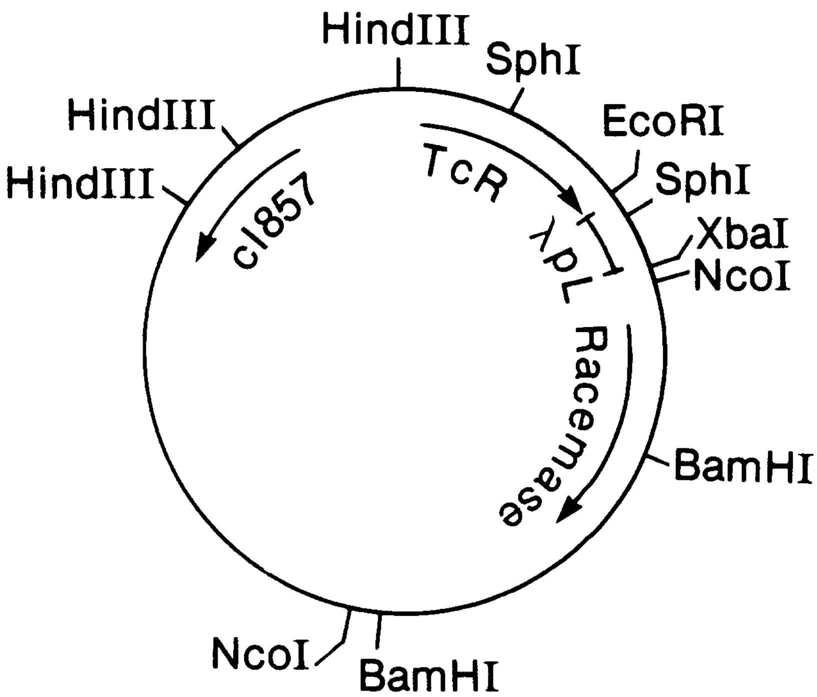

# FIG.4

## Beta-Lactam Biosynthesis

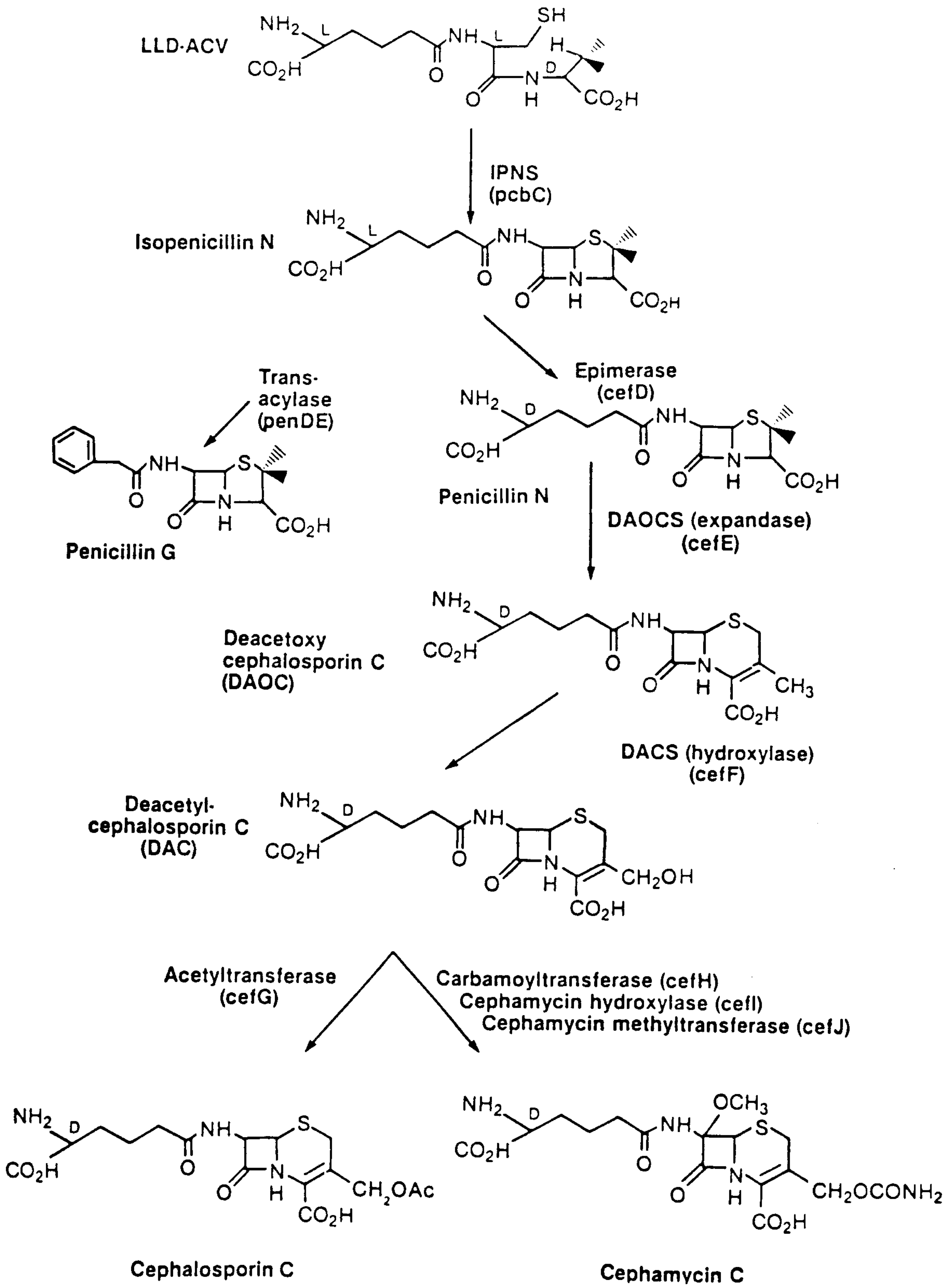


LLD-ACV

Isopenicillin N

Penicillin N

Penicillin G

Deacetoxy cephalosporin C (DAOC)

Deacetyl-cephalosporin C (DAC)

Cephalosporin C

Cephamycin C

FIG.5

**Restriction Site and Function Map of Plasmid pPSJ78 (~14.5 kb)**

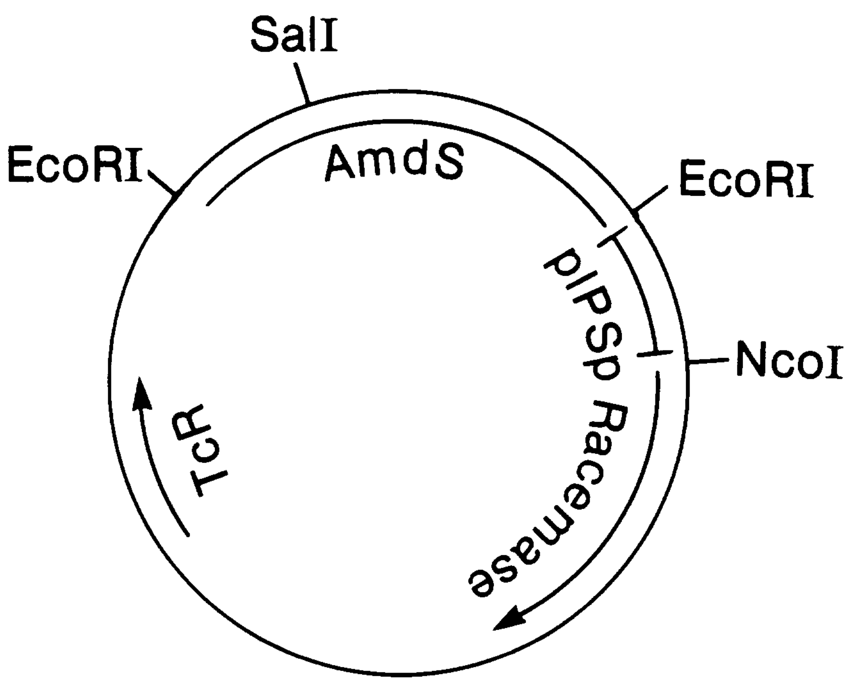

FIG.6

**Restriction Site and Function Map of Plasmid pLC2 (7.05 kb)**

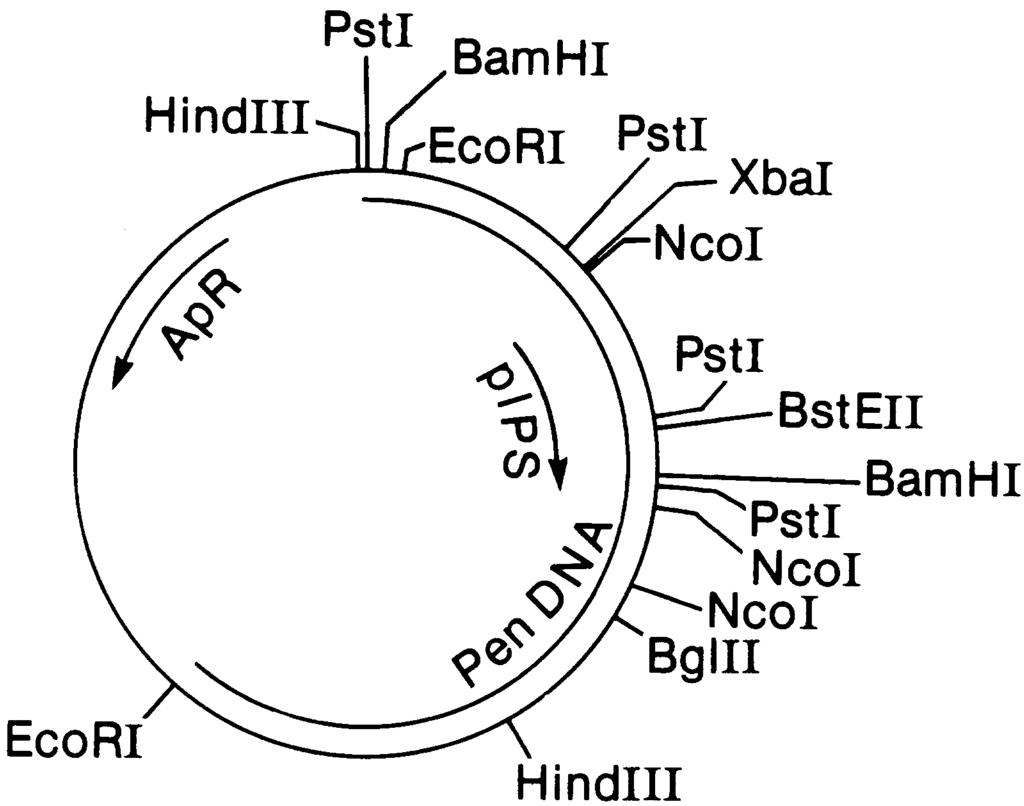

FIG.7

**Restriction Site and Function Map of Plasmid pPSJ77 (~ 8.8 kb)**

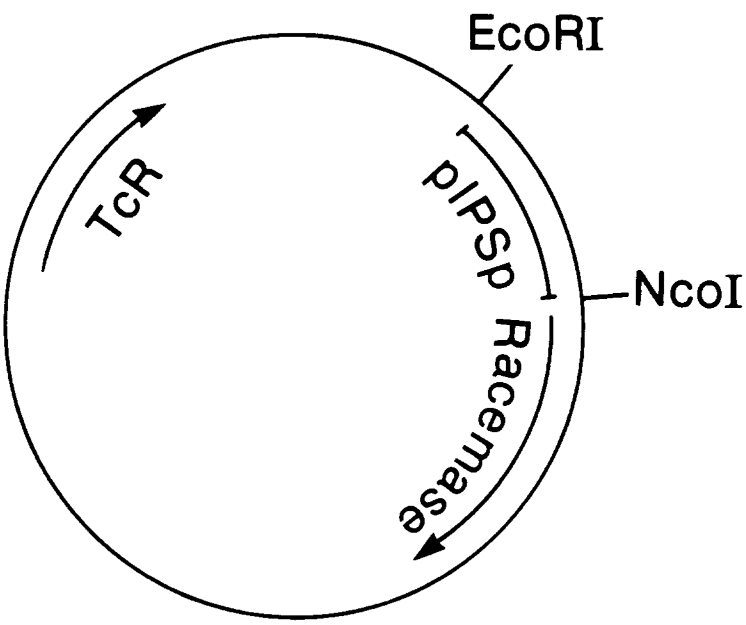

FIG.8
**Restriction Site and Function Map of Plasmid pPS51 (7661 bp)**

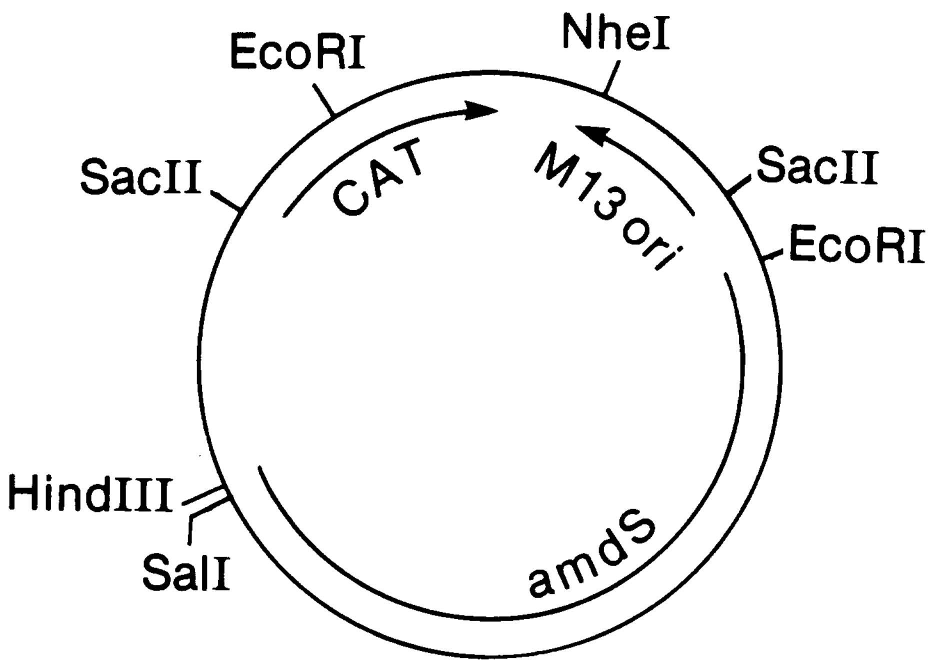

FIG.9

**Restriction Site and Function Map of Plasmid pMLC12**

**(2671 bp)**

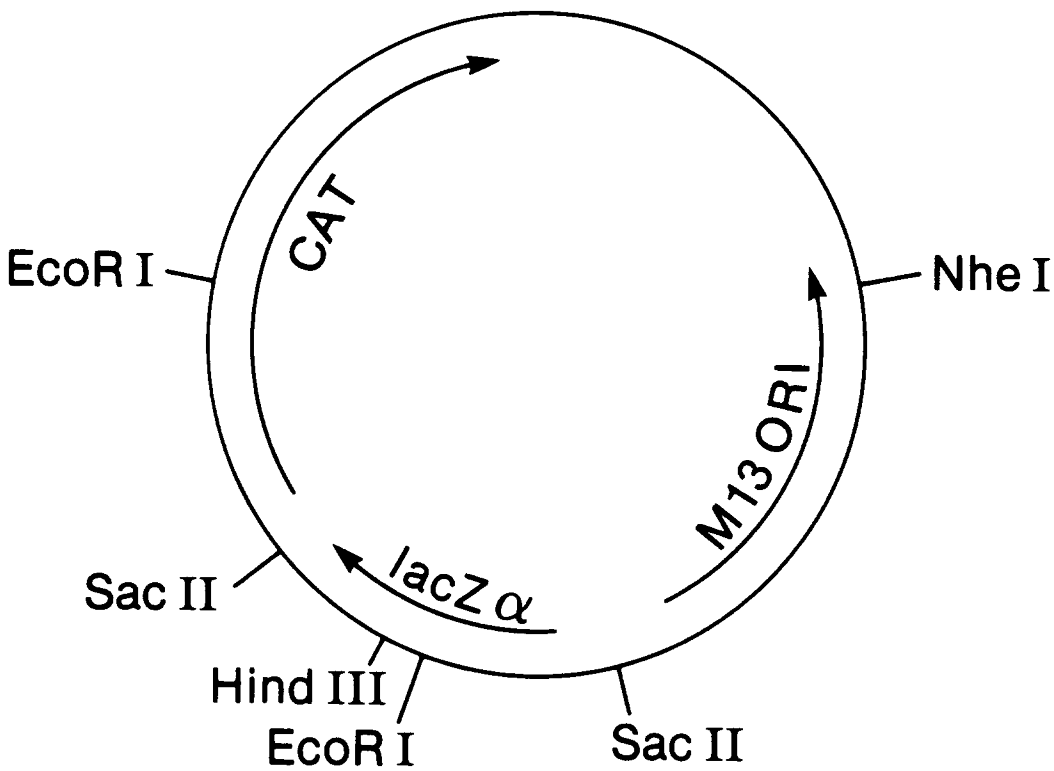

FIG.10
**Restriction Site and Function Map of Plasmid p3SR2**
**(9362 bp)**